(19) Europäisches Patentamt
European Patent Office
Office européen des brevets

(11) EP 4 538 278 A1

(12) **EUROPEAN PATENT APPLICATION**
published in accordance with Art. 153(4) EPC

(43) Date of publication:
**16.04.2025 Bulletin 2025/16**

(21) Application number: **23820101.6**

(22) Date of filing: **08.06.2023**

(51) International Patent Classification (IPC):
***C07F 5/02** (2006.01)* ***A61K 31/69** (2006.01)*
***A61P 35/00** (2006.01)*

(52) Cooperative Patent Classification (CPC):
**A61K 31/69; A61P 35/00; C07F 5/02**

(86) International application number:
**PCT/KR2023/007804**

(87) International publication number:
**WO 2023/239166 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.06.2022 KR 20220069262**

(71) Applicant: **Daewoong Pharmaceutical Co., Ltd.**
**Hwaseong-si, Gyenoggi-do 18623 (KR)**

(72) Inventors:
- **KIM, Wol Young**
  **Seoul 06170 (KR)**
- **JEONG, Seung Jae**
  **Seoul 06170 (KR)**
- **KIM, Ji Duck**
  **Seoul 06170 (KR)**
- **KIM, Eungi**
  **Seoul 06170 (KR)**
- **SHIN, Iljin**
  **Seoul 06170 (KR)**
- **PARK, Joon Seok**
  **Seoul 06170 (KR)**

(74) Representative: **Germain Maureau**
**12, rue Boileau**
**69006 Lyon (FR)**

(54) **ARGINASE INHIBITOR AND PHARMACEUTICAL COMPOSITION COMPRISING SAME**

(57) The present disclosure relates to a compound represented by Chemical Formula 1 in the present specification, or a pharmaceutically acceptable salt thereof, and a pharmaceutical composition comprising the same can be used favorably for preventing or treating cancer or tumors.

EP 4 538 278 A1

## Description

### [TECHNICAL FIELD]

The present disclosure relates to a compound having a novel structure that can be used favorably for preventing or treating cancer or tumors.

### [BACKGROUND ART]

In relation to the development of immune anticancer agents, which are currently emerging in the field of development of cancer therapeutic agents, it is a well-known fact that arginine depletion in the tumor microenvironment due to elevated levels of arginase is observed in cancer patients. In fact, such mechanisms are known to allow tumor cells of acute myeloid leukemia, breast cancer, prostate cancer, glioblastoma, esophageal cancer, renal cell carcinoma, and the like to avoid being destroyed by the body's immune system. Therefore, there has emerged a need to develop arginase inhibitors that inhibit arginase to restore arginine levels in the tumor microenvironment, and promote tumor-killing activity of cytotoxic T cells.

In relation to arginase inhibitors, pyrrolidine-based arginase inhibitors have been developed and known to date, but there is a demand to develop arginase inhibitors having new chemical structures.

Therefore, the present inventors have studied novel compounds having chemical structures different from those of arginase inhibitors reported to date, and as a result, have confirmed that compounds having a bicyclic structure of octahydropyrrolo[3,4-b]pyrrole as the mother nucleus have excellent arginase inhibitory activity, thereby completing the present disclosure.

### [DETAILED DESCRIPTION OF THE INVENTION]

### [Technical Problem]

It is an object of the present disclosure to provide a compound having a novel structure that can be used favorably for preventing or treating cancer or tumors.

### [Technical Solution]

In order to achieve the above object, provided herein is a compound represented by the following Chemical Formula 1, or a pharmaceutically acceptable salt thereof:

[Chemical Formula 1]

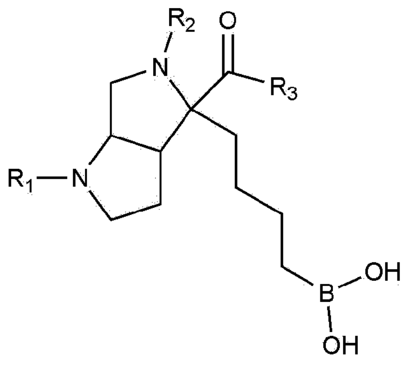

in Chemical Formula 1,

$R_1$ and $R_2$ are each independently hydrogen; a $C_{1-4}$ alkyl which is unsubstituted or substituted with amino, ($C_{1-4}$ alkyl) amino, or di($C_{1-4}$ alkyl)amino; or - $L_1$-$R'_1$,

$L_1$ is -CO-, -CO-O-, -(CO)-O-($C_{1-4}$ alkylene)-O-(CO)-, or -$CH_2$-O-CO-,
$R'_1$ is -CH($NH_2$)-$R_A$, -CH(N($CH_3$)$_2$)-$R_A$, $R_B$, or Rc,

$R_3$ is hydroxy, or -$L_3$-$R'_3$,

$L_3$ is -NH-, -O-, or $-O-(CH_2)_{n3}-O-CO-$,
$R'_3$ is $-CH(COOH)-R_A$, $-CH(NH_2)-R_A$, $R_B$, or Rc,

each $R_A$ is independently hydrogen; or a $C_{1-6}$ alkyl which is unsubstituted or substituted with hydroxy, mercapto, hydroseleno, guanidino, amino, carboxy, carbamoyl, $C_{1-6}$ alkylthio, phenyl, hydroxyphenyl, or $C_{4-10}$ heteroaryl containing one or two N,
each $R_B$ is independently a $C_{4-10}$ heterocycloalkyl containing N,
each Rc is independently a $C_{1-6}$ alkyl group which is unsubstituted or substituted with a $C_{6-10}$ aryl or a $C_{4-10}$ heterocycloalkyl containing N; a $C_{6-10}$ aryl which is unsubstituted or substituted with a $C_{1-6}$ alkoxy; or a $C_{3-6}$ cycloalkyl, and
n3 is an integer of 1 to 3.

Preferably, $R_1$ and $R_2$ are each independently hydrogen, methyl, methylaminoethyl, or a substituent represented by any one of the following:

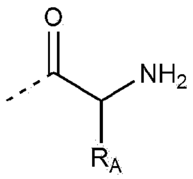
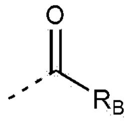
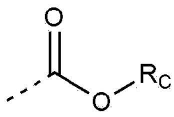
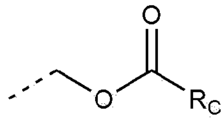
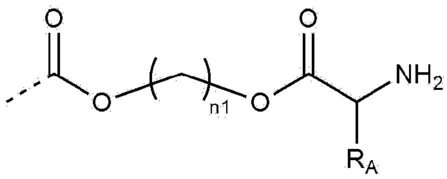
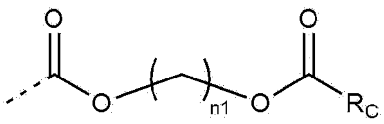
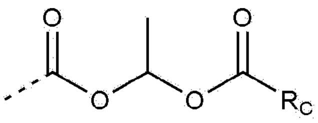
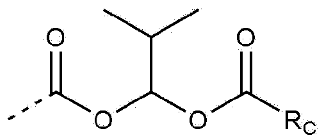

wherein, $R_A$, $R_B$, and Rc are as defined above, and n1 is an integer of 1 to 3.
Preferably, $R_3$ is hydroxy, or a substituent represented by any one of the following:

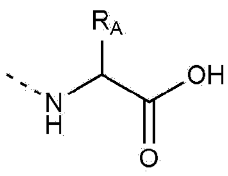
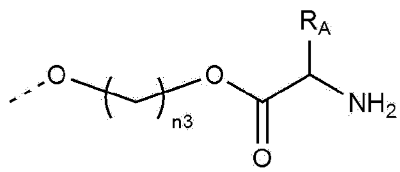
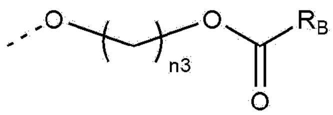
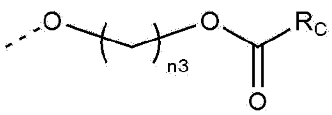
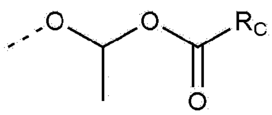
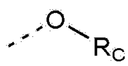

wherein, $R_A$, $R_B$, Rc and n3 are as defined above.
Preferably, each $R_A$ is independently hydrogen; or a $C_{1-6}$ alkyl which is unsubstituted or substituted with hydroxy, mercapto, hydroseleno, guanidino, amino, carboxy, carbamoyl, methylthio, phenyl, hydroxyphenyl, indole, or imidazole. More preferably, each $R_A$ is independently hydrogen, methyl, ethyl, 1-hydroxy-1-ethyl, propyl, isopropyl, normal butyl,

isobutyl, tertbutyl, or benzyl.

Preferably, each $R_A$ is independently a residue of a natural amino acid, wherein the residue of the natural amino acid means a structure excluding the terminal $-CH(NH_2)(COOH)$. For example, if A is an alanine residue, it means methyl. Preferably, the natural amino acid is arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, or tryptophan.

Preferably, $R_B$ is pyrrolidinyl.

Preferably, each Rc is independently a $C_{1-6}$ alkyl which is unsubstituted or substituted with phenyl, or piperidinyl; or a $C_{6-10}$ aryl which is unsubstituted or substituted with methoxy. More preferably, each Rc is independently methyl, pyrrolidinylmethyl, ethyl, propyl, isopropyl, normal butyl, isobutyl, tertbutyl, phenyl, methoxyphenyl, benzyl, or cyclohexyl.

Preferably, $R_1$ is hydrogen, and $R_2$ is hydrogen, or

$R_1$ is hydrogen, and $R_3$ is hydroxy, or
$R_2$ is hydrogen, and $R_3$ is hydroxy.

Preferably, the Chemical Formula 1 is represented by any one of the following Chemical Formulas 2 to 5:

[Chemical Formula 2]

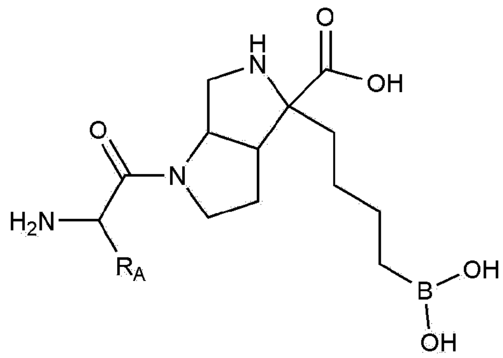

[Chemical Formula 3]

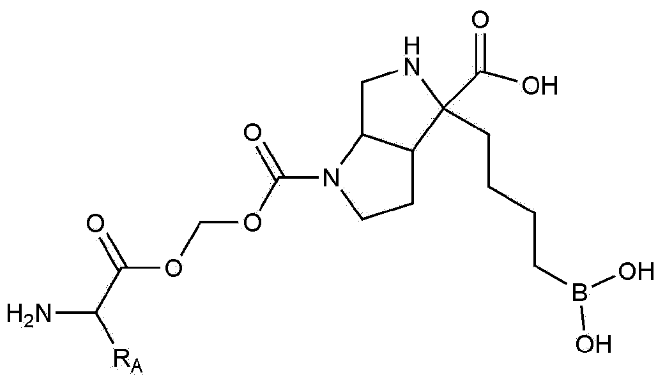

[Chemical Formula 4]

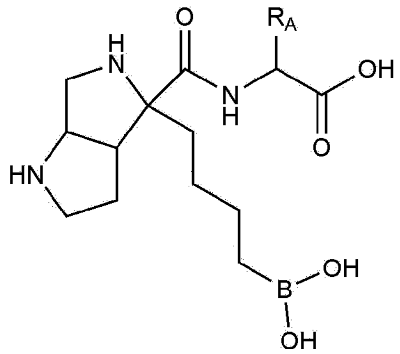

[Chemical Formula 5]

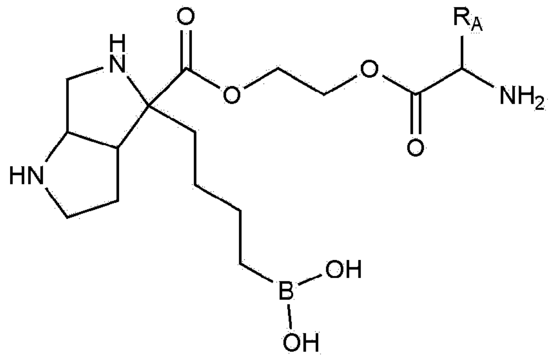

in Chemical Formulas 2 to 5,
$R_A$ is as defined above.

Representative examples of the compounds represented by Chemical Formula 1 are as follows:

1) (3aS,4R,6aR)-4-(4-boronobutyl)octahydropyrrolo[2,3-c]pyrrole-4-carboxylic acid,
2) (3aS,4R,6aR)-1-((S)-2-aminopropanoyl)-4-(4-boronobutyl)octahydropyrrolo[3,4-b]pyrrole-4-carboxylic acid,
3) (3aS,4R,6aR)-1-((S)-2-amino-3-phenylpropanoyl)-4-(4-boronobutyl)octahydropyrrolo[3,4-b]pyrrole-4-carboxylic acid,
4) (3aS,4R,6aR)-4-(4-boronobutyl)-1-((S)-pyrrolidine-2-carbonyl)octahydropyrrolo[3,4-b]pyrrole-4-carboxylic acid,
5) (3aS,4R,6aR)-1-((2R,3S)-2-amino-3-hydroxybutanoyl)-4-(4-boronobutyl)octahydropyrrolo[3,4-b]pyrrole-4-carboxylic acid,
6) (3aS,4R,6aR)-1-((S)-2-amino-3-methylbutanoyl)-4-(4-boronobutyl)octahydropyrrolo[3,4-b]pyrrole-4-carboxylic acid,
7) (3aS,4R,6aR)-1-((S)-2-amino-4-methylpentanoyl)-4-(4-boronobutyl)octahydropyrrolo[3,4-b]pyrrole-4-carboxylic acid,
8) (3aS,4R,6aR)-1-((2S,3S)-2-amino-3-methylpentanoyl)-4-(4-boronobutyl)octahydropyrrolo[3,4-b]pyrrole-4-carboxylic acid,
9) (3aS,4R,6aR)-1-((R)-2-aminopropanoyl)-4-(4-boronobutyl)octahydropyrrolo[3,4-b]pyrrole-4-carboxylic acid,
10) 4-((3aS,4R,6aR)-4-(ethoxycarbonyl)octahydropyrrolo[2,3-c]pyrrol-4-yl)butylboronic acid,
11) 4-((3aS,4R,6aR)-4-(isopropoxycarbonyl)octahydropyrrolo[2,3-c]pyrrol-4-yl)butylboronic acid,
12) 4-((3aS,4R,6aR)-4-(((isopropoxycarbonyloxy)methoxy)carbonyl)octahydropyrrolo[3,4-b]pyrrol-4-yl)butylboronic acid,
13) 4-((3aS,4R,6aR)-4-(((tert-butoxycarbonyloxy)methoxy)carbonyl)octahydropyrrolo[3,4-b]pyrrol-4-yl)butylboronic acid,
14) 4-((3aS,4R,6aR)-4-((1-(ethoxycarbonyloxy)ethoxy)carbonyl)octahydropyrrolo[3,4-b]pyrrol-4-yl)butylboronic acid,
15) 4-((3aS,4R,6aR)-4-((1-(cyclohexyloxycarbonyloxy)ethoxy)carbonyl)octahydropyrrolo[3,4-b]pyrrol-4-yl)butylboronic acid,

16) 4-((3aS,4R,6aR)-4-((1-(isopropoxycarbonyloxy)ethoxy)carbonyl)octahydropyrrolo[3,4-b]pyrrol-4-yl)butylboronic acid,
17) 4-((3aS,4R,6aR)-4-((1-(pivaloyloxy)ethoxy)carbonyl)octahydropyrrolo[3,4-b]pyrrol-4-yl)butylboronic acid,
18) 4-((3aS,4R,6aR)-4-((2-(pivaloyloxy)ethoxy)carbonyl)octahydropyrrolo[3,4-b]pyrrol-4-yl)butylboronic acid,
19) (3aS,4R,6aR)-4-(4-boronobutyl)-1-((1-(cyclohexanecarbonyloxy)ethoxy)carbonyl)octahydropyrrolo[3,4-b]pyrrole-4-carboxylic acid,
20) (3aS,4R,6aR)-4-(4-boronobutyl)-1-((1-(pentanoyloxy)ethoxy)carbonyl)octahydropyrrolo[3,4-b]pyrrole-4-carboxylic acid,
21) (3aS,4R,6aR)-4-(4-boronobutyl)-1-((1-(pivaloyloxy)ethoxy)carbonyl)octahydropyrrolo[2,3-c]pyrrole-4-carboxylic acid,
22) (3aS,4R,6aR)-4-(4-boronobutyl)-1-((1-((S)-2-(dimethylamino)-3-methylbutanoyloxy)ethoxy)carbonyl)octahydropyrrolo[3,4-b]pyrrole-4-carboxylic acid,
23) (3aS,4R,6aR)-4-(4-boronobutyl)-1-((1-(cyclohexanecarbonyloxy)-2-methylpropoxy)carbonyl)octahydropyrrolo[3,4-b]pyrrole-4-carboxylic acid,
24) (3aS,4R,6aR)-4-(4-boronobutyl)-1-((2-methyl-1-(pivaloyloxy)propoxy)carbonyl)octahydropyrrolo[3,4-b]pyrrole-4-carboxylic acid,
25) (3aS,4R,6aR)-4-(4-boronobutyl)-1-(ethoxycarbonyl)octahydropyrrolo[3,4-b]pyrrole-4-carboxylic acid,
26) (3aS,4R,6aR)-4-(4-boronobutyl)-1-(isobutoxycarbonyl)octahydropyrrolo[3,4-b]pyrrole-4-carboxylic acid,
27) (3aS,4R,6aR)-4-(4-boronobutyl)-1-methyloctahydropyrrolo[3,4-b]pyrrole-4-carboxylic acid,
28) (3aS,4R,6aR)-4-(4-boronobutyl)-1-(2-(methylamino)ethyl)octahydropyrrolo[2,3-c]pyrrole-4-carboxylic acid,
29) (3aS,4R,6aR)-4-(4-boronobutyl)-1-(butyryloxymethyl)octahydropyrrolo[3,4-b]pyrrole-4-carboxylic acid,
30) (3aS,4R,6aR)-1-((acetoxymethoxy)carbonyl)-4-(4-boronobutyl)octahydropyrrolo[3,4-b]pyrrole-4-carboxylic acid,
31) (S)-2-((3aS,4R,6aR)-4-(4-boronobutyl)octahydropyrrolo[3,4-b]pyrrole-4-carboxamido)propanoic acid,
32) (S)-2-((3aS,4R,6aR)-4-(4-boronobutyl)octahydropyrrolo[3,4-b]pyrrole-4-carboxamido)-3-methylbutanoic acid,
33) (S)-2-((3aS,4R,6aR)-4-(4-boronobutyl)octahydropyrrolo[2,3-c]pyrrole-4-carboxamido)-4-methylpentanoic acid,
34) (2S,3R)-2-((3aS,4R,6aR)-4-(4-boronobutyl)octahydropyrrolo[3,4-b]pyrrole-4-carboxamido)-3-methylpentanoic acid,
35) (S)-2-((3aS,4R,6aR)-4-(4-boronobutyl)octahydropyrrolo[3,4-b]pyrrole-4-carboxamido)-3-phenylpropanoic acid,
36) 4-((3aS,4R,6aR)-4-((2-((S)-2-aminopropanoyloxy)ethoxy)carbonyl)octahydropyrrolo[2,3-c]pyrrol-4-yl)butylboronic acid,
37) 4-((3aS,4R,6aR)-4-((2-((S)-2-amino-3-methylbutanoyloxy)ethoxy)carbonyl)octahydropyrrolo[3,4-b]pyrrol-4-yl)butylboronic acid,
38) 4-((3aS,4R,6aR)-4-((2-((2S,3S)-2-amino-3-methylpentanoyloxy)ethoxy)carbonyl)octahydropyrrolo[2,3-c]pyrrol-4-yl)butylboronic acid,
39) 4-((3aS,4R,6aR)-4-((2-((S)-2-amino-4-methylpentanoyloxy)ethoxy)carbonyl)octahydropyrrolo[2,3-c]pyrrol-4-yl)butylboronic acid,
40) 4-((3aS,4R,6aR)-4-((2-((S)-2-amino-3-phenylpropanoyloxy)ethoxy)carbonyl)octahydropyrrolo[2,3-c]pyrrol-4-yl)butylboronic acid,
41) 4-((3aS,4R,6aR)-4-((2-((S)-pyrrolidine-2-carbonyloxy)ethoxy)carbonyl)octahydropyrrolo[3,4-b]pyrrol-4-yl)butylboronic acid,
42) 4-((3aS,4R,6aR)-4-((propionyloxymethoxy)carbonyl)octahydropyrrolo[2,3-c]pyrrol-4-yl)butylboronic acid,
43) 4-((3aS,4R,6aR)-4-((isobutyryloxymethoxy)carbonyl)octahydropyrrolo[3,4-b]pyrrol-4-yl)butylboronic acid,
44) 4-((3aS,4R,6aR)-4-(benzyloxycarbonyl)octahydropyrrolo[3,4-b]pyrrol-4-yl)butylboronic acid,
45) 4-((3aS,4R,6aR)-4-((piperidin-4-ylmethoxy)carbonyl)octahydropyrrolo[3,4-b]pyrrol-4-yl)butylboronic acid,
46) 4-((3aS,4R,6aR)-4-(phenoxycarbonyl)octahydropyrrolo[3,4-b]pyrrol-4-yl)butylboronic acid,
47) 4-((3aS,4R,6aR)-4-((2-methoxyphenoxy)carbonyl)octahydropyrrolo[3,4-b]pyrrol-4-yl)butylboronic acid,
48) (3aS,4R,6aR)-1-((((S)-2-amino-3-phenylpropanoyloxy)methoxy)carbonyl)-4-(4-boronobutyl)octahydropyrrolo[3,4-b]pyrrole-4-carboxylic acid,
49) (3aS,4R,6aR)-1-((((S)-2-aminopropanoyloxy)methoxy)carbonyl)-4-(4-boronobutyl)octahydropyrrolo[3,4-b]pyrrole-4-carboxylic acid,
50) (3aS,4R,6aR)-4-(4-boronobutyl)-1-((((S)-pyrrolidine-2-carbonyloxy)methoxy)carbonyl)octahydropyrrolo[3,4-b]pyrrole-4-carboxylic acid,
51) (3aS,4R,6aR)-1-((((2S,3R)-2-amino-3-hydroxybutanoyloxy)methoxy)carbonyl)-4-(4-boronobutyl)octahydropyrrolo[3,4-b]pyrrole-4-carboxylic acid,
52) (3aS,4R,6aR)-1-((((S)-2-amino-3-methylbutanoyloxy)methoxy)carbonyl)-4-(4-boronobutyl)octahydropyrrolo[3,4-b]pyrrole-4-carboxylic acid,
53) (3aS,4R,6aR)-1-((((S)-2-amino-4-methylpentanoyloxy)methoxy)carbonyl)-4-(4-boronobutyl)octahydropyrrolo[2,3-c]pyrrole-4-carboxylic acid, and
54) (3aS,4R,6aR)-1-((((2S,3S)-2-amino-3-methylpentanoyloxy)methoxy)carbonyl)-4-(4-boronobutyl)octahydropyrrolo[3,4-b]pyrrole-4-carboxylic acid.

In addition, the compounds of the present disclosure may exist in the form of salts, especially pharmaceutically acceptable salts. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

Further, a pharmaceutically unacceptable salt or solvate of the compound represented by Chemical Formula 1 may be used as an intermediate when preparing the compound represented by Chemical Formula 1, or the pharmaceutically acceptable salt or the solvate thereof.

Further, the compound of Chemical Formula 1 according to the present disclosure includes not only pharmaceutically acceptable salts thereof, but also solvates such as hydrates that can be prepared therefrom, and includes all possible stereoisomers, without being limited thereto. The solvate and the stereoisomer of the compound of Chemical Formula 1 may be prepared from the compound of Chemical Formula 1 using common methods known in the art.

In addition, the compound of Chemical Formula 1 according to the present invention may be prepared either in a crystalline form or in a non-crystalline form, and when the compound of Chemical Formula 1 is prepared in a crystalline form, it may be optionally hydrated or solvated. In the present disclosure, the compound of Chemical Formula 1 may not only include a stoichiometric hydrate, but also include a compound containing various amounts of water. The solvate of the compound of Chemical Formula 1 according to the present disclosure includes both stoichiometric solvates and non-stoichiometric solvates.

Furthermore, as an example, the compound represented by Chemical Formula 1 according to the present disclosure can be prepared through Reaction Scheme 1 below.

[Reaction Scheme 1]

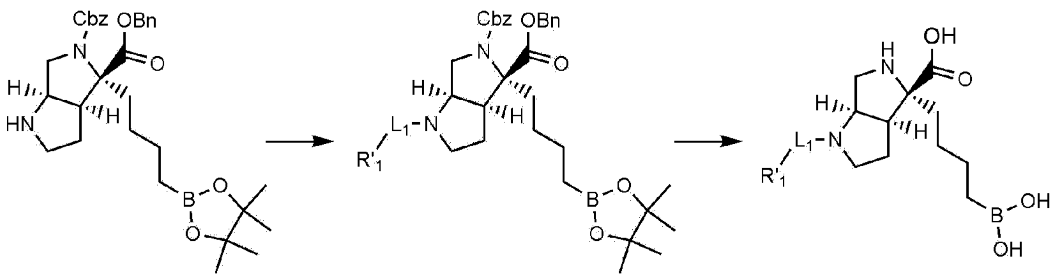

The Reaction Scheme 1 illustrates the case where $R_1$ is $-L_1-R'_1$, $R_2$ is hydrogen, and $R_3$ is hydroxy, and can be applied to the preparation of the remaining compounds represented by Chemical Formula 1 other than the above. The first reaction of the Reaction Scheme 1 is a $-L_1-R'_1$ substitution reaction, and the second reaction is a protecting group removal reaction. The preparation method can be more specifically embodied in Preparation Examples provided hereinafter.

In addition, the present disclosure provides any one compound selected from the group consisting of the following, wherein the following compounds can be used as intermediates in the preparation of the compound represented by Chemical Formula 1 according to the present disclosure.

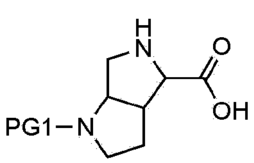 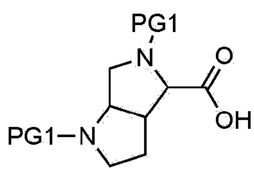 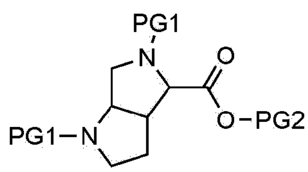

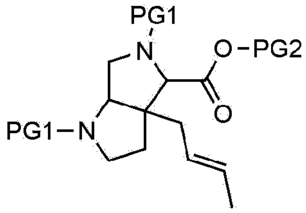 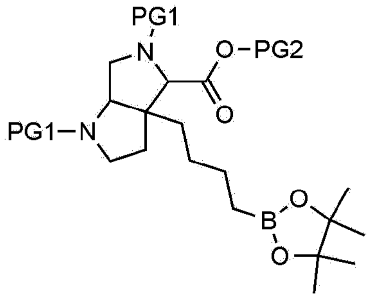

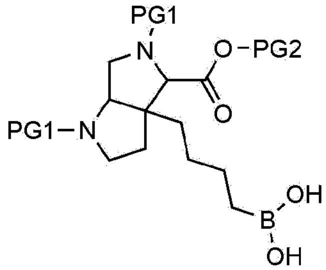 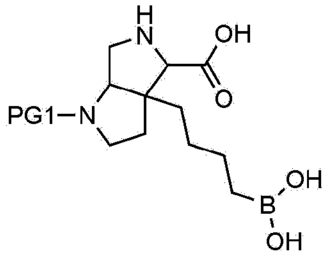

wherein,

PG1 and PG2 are each independently identical or different, and represents a protecting group.

The protecting group is not particularly limited as long as it is widely used in the technical field to which the present disclosure belongs. Preferably, PG1 is an amine protecting group, and PG2 is a carboxylic acid protecting group. Representative examples of PG1 include carbobenzyloxy, tert-butoxycarbonyl, p-methoxybenzylcarbonyl, acetyl, benzoyl, benzyl, p-methoxybenzyl, p-methoxyphenyl, and the like, but are not limited thereto. Representative examples of PG2 include acetyl, benzoyl, benzyl, methoxyethoxymethyl ether, dimethoxytrityl, methoxymethyl ether, methoxytrityl ((4-methoxyphenyl)diphenylmethyl), p-methoxybenzyl ether, p-methoxyphenyl ether, methylthiomethyl ether, pivaloyl, tert-butyl ether, tetrahydropyranyl, tetrahydrofuran, trityl, trimethylsilyl, tert-butyldimethylsilyl, and the like.

Preferably, the compound is any one selected from the group consisting of the following:

(3aR,4R,6aR)-1-(tert-butoxycarbonyl)octahydropyrrolo[3,4-b]pyrrole-4-carboxylic acid,
(3aS,4R,6aR)-5-((benzyloxy)carbonyl)-1-(tert-butoxycarbonyl)octahydropyrrolo[3,4-b]pyrrole-4-carboxylic acid,
4,5-dibenzyl 1-(tert-butyl) (3aS,4R,6aR)-hexahydropyrrolo[3,4-b]pyrrole-1,4,5-tricarboxylate,
4,5-dibenzyl 1-(tert-butyl) (3aS,4R,6aR)-4-((E)-but-2-en-1-yl)hexahydropyrrolo[3,4-b]pyrrole-1,4,5-tricarboxylate,
4,5-dibenzyl 1-(tert-butyl)(3aS,4R,6aR)-4-(4-(4,4,5,5-tetramethyl-1,3,2-dioxaborolan-2-yl)butyl)hexahydropyrrolo[3,4-b]pyrrole-1,4,5-tricarboxylate,
(4-((3aS,4R,6aR)-4,5-bis((benzyloxy)carbonyl)-1-(tert-butoxycarbonyl)octahydropyrrolo[3,4-b]pyrrole-4-yl)butyl) boronic acid, and
(3aS,4R,6aR)-5-(benzyloxycarbonyl)-4-(4-boronobutyl)-1-(tert-butoxycarbonyl)octahydropyrrolo[3,4-b]pyrrole-4-carboxylic acid.

In addition, the compound represented by Chemical Formula 1, or a pharmaceutically acceptable salt thereof has an arginase inhibitory effect, and can inhibit arginase to restore arginine levels in the tumor microenvironment, and promote tumor-killing activity of cytotoxic T cells. Therefore, the present disclosure provides a pharmaceutical composition for preventing or treating cancer or tumors, comprising the compound represented by Chemical Formula 1, or a pharmaceutically acceptable salt thereof.

As used herein, the term "prevention" refers to any act to delay or inhibit occurrence, spread or recurrence of the above-mentioned diseases by administration of the composition of the present disclosure, and "treatment" refers to any act to improve or change the symptoms of the above diseases for the better by administration of the composition of the present disclosure.

The pharmaceutical composition according to the present disclosure can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present disclosure can be dissolved in

oils, propylene glycol or other solvents commonly used in the preparation of injection solutions. Furthermore, the compounds of the present disclosure can be formulated in ointments or creams for topical application.

A preferred dose of the compound of the present disclosure may be varied according to the condition and weight of a patient, the severity of a disease, the type of a drug, and the route and duration of administration, but it may be suitably selected by those skilled in the art. In order to achieve the desirable effects, however, the compound of the present disclosure may be administrated daily at a dose of 0.0001 to 100 mg/kg (body weight), and preferably 0.001 to 100 mg/kg (body weight). The administration may be performed once a day or in divided doses each day through an oral or parenteral route.

Depending on the method of administration, the pharmaceutical composition may contain the compound of the present disclosure in an amount of 0.001 to 99% by weight, preferably 0.01 to 60% by weight.

The pharmaceutical composition according to the present disclosure may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

## [Advantageous Effects]

A compound represented by Chemical Formula 1 of the present disclosure, or a pharmaceutically acceptable salt thereof can be used favorably for preventing or treating cancer or tumors.

## [DETAILED DESCRIPTION OF THE EMBODIMENTS]

Hereinafter, preferred examples are presented to assist in the understanding of the present disclosure. However, the following examples are for illustrative purposes only, and should not be construed as limiting the scope of the present disclosure to these examples.

### Example 1: Preparation of (3aS,4R,6aR)-4-(4-boronobutyl)octahydropyrrolo[3,4-b]pyrrole-4-carboxylic acid dihydrochloride

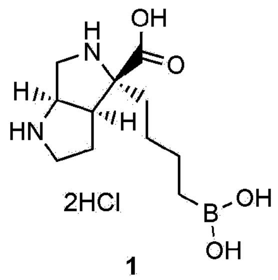

1

### Step 1) Preparation of Dibenzyl (R)-4-oxopyrrolidine-1,2-dicarboxylate

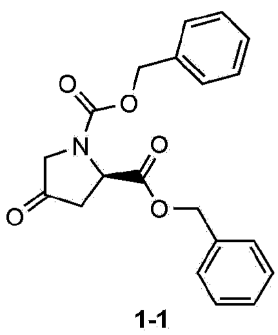

1-1

Methylene chloride (1.5 L) was cooled to -78°C, and then oxalyl chloride (90.0 ml) and dimethyl sulfoxide (92.7

ml) were slowly added dropwise. The mixture was stirred at -78°C for 10 minutes, and then dibenzyl (2R,4R)-4-hydroxypyrrolidine-1,2-dicarboxylate (186.0 g) was dissolved in methylene chloride (700 ml), and slowly added dropwise at -78°C. The solution was stirred for 30 minutes, and then triethylamine (255 ml) was slowly added dropwise at -78°C. It was stirred at -78°C for 1 hour to complete the reaction. The reaction solution was added to a 1N-HCl solution at 0~5°C, and then the organic layer was separated. The aqueous layer was extracted twice using methylene chloride (1 L), and the organic layer was separated. The organic layer was dried over $MgSO_4$ and concentrated. The product was subjected to a column separation using ethyl acetate (in hexane 30%) to obtain Compound 1-1 (117 g, yield: 63%).

MS: $[M+H]^+$ = 354
$^1H$ NMR(500 MHz, $CDCl_3$) 7.38-7.25 (m, 10H), 5.26-5.09 (m, 4H), 4.96-4.86 (m, 1H), 4.05-3.95 (m, 2H), 3.01-2.92 (m, 1H), 2.63-2.59 (m, 1H)

**Step 2) Preparation of 4,5-Dibenzyl 1-(tert-butyl) (R)-4,6-dihydropyrrolo[3,4-b]pyrrole-1,4,5-tricarboxylate**

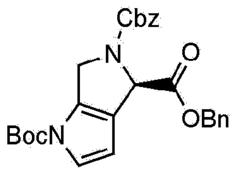

**1-2**

Compound 1-1 (67 g) was dissolved in tetrahydrofuran (300 ml), and then cooled to -78°C under nitrogen. 2.5N n-BuLi (84 ml) was slowly added dropwise thereto at -78°C under nitrogen. The solution was stirred at -50 to -55°C for 1 hour, and then cooled to -78°C. Tert-butyl (2-oxoethyl)carbamate (34 g) was dissolved in tetrahydrofuran (100 ml), and then slowly added dropwise at -78°C under nitrogen. It was stirred at -50 to -55°C for 1 hour to complete the reaction. The reaction solution was added to a saturated $NH_4Cl$ solution at 0~5°C, and then the layers were separated using ethyl acetate (1.4 L) and the organic layer was separated. The aqueous layer was extracted twice using ethyl acetate (670 ml) and the organic layer was separated. The organic layer was dried over $MgSO_4$ and concentrated. It was dissolved in methylene chloride (1 L), and then conc-HCl solution (8.1 ml) was slowly added dropwise. It was stirred at room temperature for 12 hours to complete the reaction. The reaction solution was cooled to 0~5°C, and then 3% $NaHCO_3$ aqueous solution (500 ml) was added thereto. The organic layer was then separated, dried over $MgSO_4$, and concentrated. The product was subjected to a column separation using ethyl acetate (in hexane 30%) to obtain Compound 1-2 (24 g, yield: 30%).

MS: $[M+H]^+$= 477
$^1H$ NMR(500 MHz,$CDCl_3$) 7.42-7.31 (m, 9H), 7.28-7.24 (m, 1H), 6.09 (m, 1H), 5.41-5.03 (m, 5H), 4.82-4.73 (m, 2H), 1.59 (m, 9H)

**Step 3) Preparation** of **(3aR,4R,6aR)-1-(tert-butoxycarbonyl)octahydropyrrolo[3,4-b]pyrrole-4-carboxylic acid**

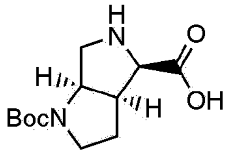

**1-3**

Compound 1-2 (24 g) was dissolved in tetrahydrofuran (500 ml) and methanol (500 ml). $Pd(OH)_2$ (12 g) was added thereto, and then stirred under a hydrogen balloon for 4 hours to complete the reaction. The reaction solution was washed with methanol, filtered through Celite, concentrated, and dried under reduced pressure to obtain Compound 1-3 (12.8 g, yield: 100%).

MS: $[M+H]^+$= 257

$^1$H NMR(500 MHz, MeOD) 4.91 (m, 1H), 4.25-4.15 (m, 1H), 3.74 (m, 1H), 3.50-3.48 (m, 2H), 3.44-3.39 (m, 1H), 2.03-1.95 (m,2H), 1.89 (m, 1H) 1.49 (s, 9H)

**Step 4) Preparation of (3aS,4R,6aR)-5-((benzyloxy)carbonyl)-1-(tert-butoxycarbonyl)octahydropyrrolo[3,4-b]pyrrole-4-carboxylic acid**

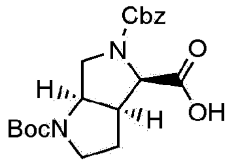

1-4

Compound 1-3 (12.8 g) was dissolved in 1 N KOH aqueous solution (100 ml), and then benzyl carbonochloridate (8.5 ml) was slowly added dropwise thereto. The solution was stirred at room temperature for 12 hours to complete the reaction. After the reaction solution was cooled to 0~5°C, ethyl acetate (150 ml) and $H_2O$ (100 ml) were added. The resulting mixture was adjusted to pH 2~3 using 6N HCl aqueous solution, the layers were separated and the organic layer was separated. Ethyl acetate (100 ml) was added twice to the aqueous layer, and the organic layer was separated, dried over $MgSO_4$, and concentrated. The product was dried under reduced pressure to obtain Compound 1-4 (20 g, yield: 100%). The obtained compound was used for the next reaction without purification.

MS: $[M+H]^+$= 391
$^1$H NMR(500 MHz, $CDCl_3$) 7.38-7.28 (m, 5H), 5.20-5.10 (m, 2H), 4.61 (m, 1H), 4.58-4.30 (m, 1H), 4.16-4.05 (m, 1H), 3.58-3.50 (m, 1H), 3.49-3.32 (m, 2H), 3.22-3.15 (m, 1H), 1.95-1.94 (m, 2H), 1.53 (s, 9H)

**Step 5) Synthesis** of **4,5-Dibenzyl 1-(tert-butyl) (3aS,4R,6aR)-hexahydropyrrolo[3,4-b]pyrrole-1,4,5-tricarboxylate**

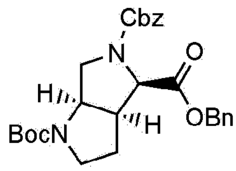

1-5

Compound 1-4 (20 g) was dissolved in N,N-dimethylformamide (100 ml). Potassium carbonate (10.42 g), sodium iodide (753 mg), and benzyl bromide (8.9 ml) were added thereto, and then stirred at room temperature for 12 hours to complete the reaction. Ethyl acetate (400 ml) and 0.5 N HCl aqueous solution (200 ml) were added to separate the layers. The organic layer was concentrated, and then subjected to a column separation with ethyl acetate (in hexane 30%) solution to obtain Compound 1-5 (20 g, yield: 83%).

MS: $[M+H]^+$= 481
$^1$H NMR(500 MHz, $CDCl_3$) 7.37-7.27 (m, 10H), 5.29-5.06 (m, 4H), 4.63-4.61 (m, 1H), 4.37-4.27 (m, 1H), 4.16-4.11 (m, 1H), 3.50-3.20 (m, 3H), 3.19-3.16 (m, 1H), 1.84 (m, 1H), 1.75 (m, 1H), 1.45 (m, 9H)

**Step 6) Synthesis of 4,5-Dibenzyl 1-(tert-butyl)(3aS,4R,6aR)-4-((E)-but-2-en-1-yl)hexahydropyrrolo[3,4-b]pyrrole-1,4,5-tricarboxylate**

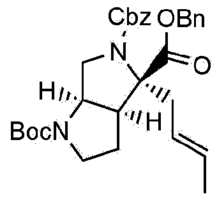

**1-6**

Compound 1-5 (20 g) was dissolved in tetrahydrofuran (200 ml), and then cooled to -78°C under nitrogen. Crotyl bromide (8.1 ml) was added dropwise, and 1N potassium bis(trimethylsilyl)amide (80 ml) was slowly added dropwise. It was stirred at -78°C for 30 minutes to complete the reaction. The reaction solution was added dropwise to a 1N HCl aqueous solution (200 ml) at 0~5°C, and extracted using ethyl acetate (200 ml). The aqueous solution was extracted twice using ethyl acetate (200 ml), and the organic layer was concentrated. The product was subjected to a column separation with ethyl acetate (in hexane 30%) to obtain Compound 1-6 (10.4 g, yield: 65%).

MS: $[M+H]^+$= 335
$^1$H NMR(500 MHz, $CDCl_3$) 5.6-4.90 (m, 6H), 4.35-3.85 (m, 2H), 3.55-3.30 (m, 1H), 3.25-3.15 (m, 2H), 2.95-2.65 (m, 3H), 1.95-2.85 (m, 1H), 1.75-1.55 (m, 4H), 1.46-1.44 (m, 9H)

**Step 7) Preparation of 4,5-Dibenzyl 1-(tert-butyl)(3aS,4R,6aR)-4-(4-(4,4,5,5-tetramethyl-1,3,2-dioxaborolan-2-yl) butyl)hexahydropyrrolo[3,4-b]pyrrole-1,4,5-tricarboxylate**

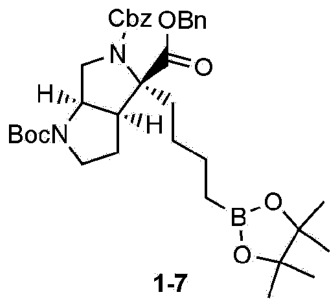

**1-7**

Chloro(1,5-cyclooctadiene)iridium(I) dimer (1.7 g) and 1,2-bis(diphenylphosphino)ethane (2.1 g) were added to methylene chloride (100 ml) under nitrogen. 4,4,5,5-Tetramethyl-1,3,2-dioxaborolane (9.66 ml) was added thereto, and then stirred for 20 minutes under nitrogen. Compound 1-6 (14 g) was dissolved in methylene chloride (50 ml), and then slowly added dropwise. It was stirred at room temperature under nitrogen for 20 hours. The reaction solution was extracted with $H_2O$ (50 ml), and the organic layer was separated. The organic layer was concentrated, and then subjected to a column separation with ethyl acetate (in hexane 30%) to obtain Compound 1-7 (8.5 g, yield: 49%).

MS: $[M+H]^+$= 663
$^1$H NMR(500 MHz, $CDCl_3$) 7.34-7.21 (m, 10H), 5.20-4.90 (m. 4H), 3.90-3.55 (m, 3H), 3.50-3.40 (m, 2H), 2.85-2.80 (m, 3H), 1.90-1.80 (m,4H), 1.42 (s, 9H), 1.35-1.30 (m, 2H), 1.20 (s, 12H), 0.6-0.8 (m, 2H)

**Step 8) Preparation of (4-((3aS,4R,6aR)-4,5-bis((benzyloxy)carbonyl)-1-(tert-butoxycarbonyl)octahydropyrrolo [3,4-b]pyrrol-4-yl)butyl)boronic acid**

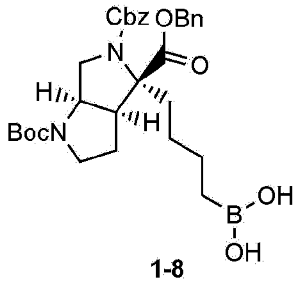

1-8

Compound 1-7 (8.5 g) was dissolved in acetone (128 ml), and then methylboronic acid (7.66 g) was added thereto. 0.1N NaOH aqueous solution (128 ml) was added thereto, and then stirred at room temperature for 12 hours. $NH_4Cl$ saturated solution (100 ml) was added, and then extracted using ethyl acetate (170 ml). The aqueous solution was extracted by adding ethyl acetate (100 ml) twice. The organic layer was concentrated, and then subjected to a column separation with methylene chloride: methanol=20:1 (v:v) to obtain Compound 1-8 (5.5 g, yield: 74%).

MS: $[M+H]^+$= 581
$^1H$ NMR(500 MHz, $CDCl_3$) 7.39-7.24 (m, 10H), 5.19-4.89 (m, 4H), 4.75-4.35 (m, 1H), 4.20-4.00 (m, 2H), 3.6-3.45 (m, 1H), 3.43-3.30 (m, 1H), 3.28-3.15 (m, 1H), 2.90-2.70 (m, 1H), 2.65-2.20 (m, 1H), 2.0-1.8 (m, 2H), 2.75-2.65 (m, 1H), 1.60-1.40 (m, 9H), 1.35-1.30 (m, 2H), 1.25-1.10 (m, 1H), 0.95-0.65 (m, 2H)

**Step 9) Preparation of (3aS,4R,6aR)-4-(4-boronobutyl)-1-(tert-butoxycarbonyl)octahydropyrrolo[3,4-b]pyrrole-4-carboxylic acid**

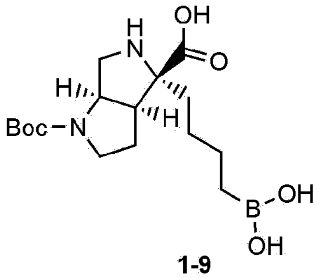

1-9

Compound 1-8 (5.5 g) was dissolved in methanol (50 ml). 10% Pd/C (2 g) was added thereto, and then stirred at room temperature for 4 hours using a hydrogen balloon to complete the reaction. The product was used directly in step 10 below.
MS: $[M+H]^+$= 357

**Step 10) Preparation of (3aS,4R,6aR)-4-(4-boronobutyl)octahydropyrrolo[3,4-b]pyrrole-4-carboxylic acid dihydrochloride**

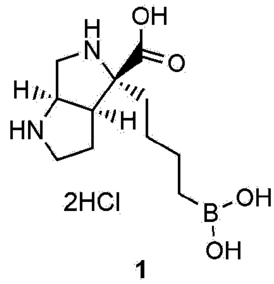

1

The product of the step 9 was washed with methanol, filtered through Celite, and concentrated. 6N HCl aqueous solution was added thereto, and stirred at room temperature for 2 hours, and concentrated. The mixture was dissolved in

$H_2O$ (5 ml), and extracted three times using diethyl ether (5 ml) to remove the organic layer. The aqueous solution was concentrated and dried under reduced pressure to obtain Compound 1 (2.5 g, yield: 80% (steps 9 and 10)).

MS: $[M+H]^+$= 257
$^1H$ NMR(500 MHz, MeOD) 4.73-4.73 (m, 1H), 3.97-3.85 (m, 2H), 3.52-3.41 (m, 1H), 3.39-3.36 (m, 2H), 2.41-2.39 (m, 1H), 2.16-2.11 (m, 2H), 2.01-1.95 (m, 1H), 1.51-1.45 (m, 3H), 1.32 (m, 1H), 0.9-0.8 (m, 2H)

**Example 2: Preparation of (3aS,4R,6aR)-1-((S)-2-aminopropanoyl)-4-(4-boronobutyl)octahydropyrrolo[3,4-b] pyrrole-4-carboxylic acid dihydrochloride**

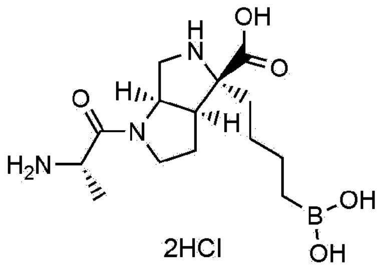

**2**

**Step 1) Preparation of Dibenzyl (3aS,4R,6aR)-4-(4-(4,4,5,5-tetramethyl-1,3,2-dioxaborolan-2-yl)butyl)hexahydropyrrolo[3,4-b]pyrrole-4,5(1H)-dicarboxylate. HCl**

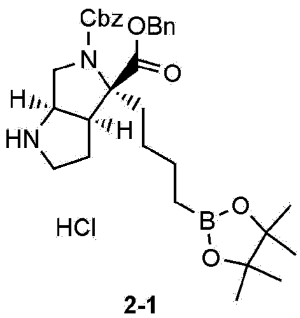

**2-1**

Compound 1-7 (800 mg) was dissolved in 1,4-dioxane (10 ml), and then 4N HCl (in dioxane; 12 ml) was added thereto. The mixture was stirred at 50°C for 1 hour to complete the reaction. The product was concentrated and dried under reduced pressure to obtain Compound 2-1 (723 mg, yield: 100%).

MS: $[M+H]^+$= 563
$^1H$ NMR(500 MHz, MeOD) 7.2-7.3 (m, 10H), 5.3-5.2 (m, 4H), 3.65-3.40 (m, 2H), 2.85-2.70 (m, 3H), 2.15-2.05 (m, 1H), 1.95-1.70 (m, 4H), 1.65-1.40 (m, 2H), 1.35-1.25 (m, 2H), 1.20 m, 2H), 0.6~0.8 (m, 2H)

**Step 2) Preparation of Dibenzyl (3aS,4R,6aR)-1-((tert-butoxycarbonyl)-L-alanyl)-4-(4-(4,4,5,5-tetramethyl-1,3,2-dioxaborolan-2-yl)butyl)hexahydropyrrolo[3,4-b]pyrrole-4,5(1H)-dicarboxylate**

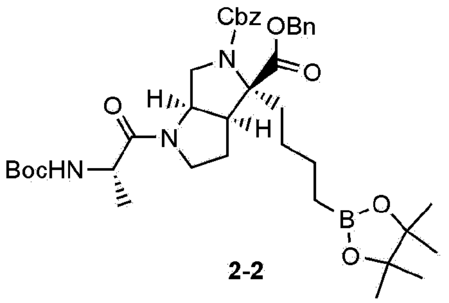

2-2

Compound 2-1 (54 mg) was dissolved in methylene chloride (1 ml). Trimethylamine (36 μL) and 2,5-dioxopyrrolidin-1-yl (tert-butoxycarbonyl)-L-alaninate (37 mg) were added thereto, and then stirred at room temperature for 12 hours to complete the reaction. After adding a saturated $NH_4Cl$ aqueous solution, the mixture was extracted, and the organic layer was separated. The organic layer was concentrated, and then subjected to a column separation using ethyl acetate (in hexane 40-50%) to obtain Compound 2-2 (47 mg, yield: 74%).

MS: $[M+H]^+$= 734
$^1H$ NMR(500 MHz, $CDCl_3$) 7.3-7.2 (m, 10H), 5.3-5.3 (m, 4H), 4.6-4.5 (m, 1H), 4.0-3.5 (m, 3H), 3.4-3.35 (m, 2H), 3.2-3.1 (m, 1H), 2.1-2.0 (m, 2H), 1.8-1.7 (m, 2H), 1.42 (s, 9H), 1.45 (m, 3H), 1.35-1.25 (m, 4H), 1.20 (s, 12H), 0.7-0.9 (m, 2H)

**Step 3) Preparation of (3aS,4R,6aR)-1-((S)-2-Aminopropanoyl)-4-(4-boronobutyl)octahydropyrrolo[3,4-b]pyrrole-4-carboxylic acid dihydrochloride**

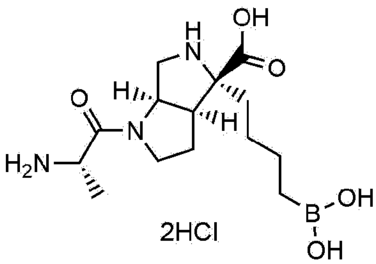

2

Compound 2 was obtained in the same manner as in steps 8 to 10 of Example 1, using Compound 2-2.

MS: $[M+H]^+$= 328, $[M-H_2O+H]^+$= 310
$^1H$ NMR(500 MHz, MeOD) 4.73 (m, 1H), 4.32-4.28 (m, 1H), 3.95-3.91 (m, 1H), 3.83-3.79 (m, 1H), 3.61-3.50 (m, 2H), 3.25-3.23 (1H), 2.30-2.29 (m, 1H), 2.13-2.07 (m, 2H), 1.98-1.96 (m, 1H), 1.33 (m, 3H), 1.31 (s, 3H), 1.30 (m, 1H), 0.84 (m, 2H)

**Example 3: Preparation of (3aS,4R,6aR)-1-((S)-2-amino-3-phenylpropanoyl)-4-(4-boronobutyl)octahydropyrrolo[3,4-b]pyrrole-4-carboxylic acid dihydrochloride**

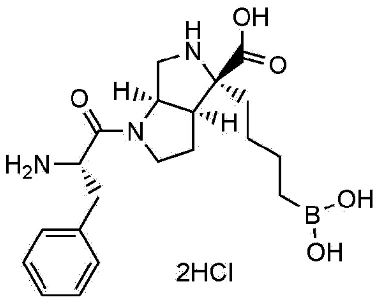

**3**

Compound 3 was obtained in the same manner as in steps 2 and 3 of Example 2, using Compound 2-1 (100 mg) and 2,5-dioxopyrrolidin-1-yl (tert-butoxycarbonyl)-L-phenylalaninate (97.8 mg).

MS: $[M+H]^+$= 368 $[M-H_2O+H]^+$= 386
$^1H$ NMR(500 MHz, MeOD) 7.40-7.29 (m, 5H), 4.89 (m, 1H), 4.64 (m, 1H), 3.81-3.79 (m, 2H), 3.55 (m, 1H), 3.12 (m, 2H), 2.99 (m, 1H), 2.58 (m, 1H), 2.06 (m, 1H), 1.95-1.90 (m, 3H), 1.48-1.44 (m, 3H), 1.44 (m, 2H)

**Example 4: Preparation of (3aS,4R,6aR)-4-(4-boronobutyl)-1-((S)-pyrrolidine-2-carbonyl)octahydropyrrolo[3,4-b]pyrrole-4-carboxylic acid dihydrochloride**

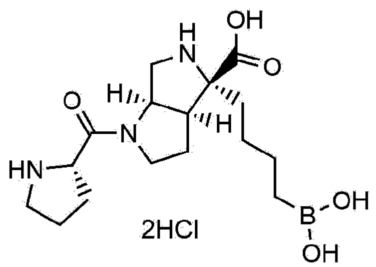

**4**

Compound 4 was obtained in the same manner as in steps 2 and 3 of Example 2, using Compound 2-1 (60 mg) and 1-(tert-butyl) 2-(2,5-dioxopyrrolidin-1-yl) (S)-pyrrolidine-1,2-dicarboxylate (40 mg).

MS: $[M+H]^+$= 354 $[M-H_2O+H]^+$= 336
$^1H$ NMR(500 MHz, MeOD) 3.70-3.60 (m, 1H), 3.55-3.30 (m, 3H), 3.10-2.70 (m, 4H), 2.20-2.0 (m, 4H), 1.95-1.70 (m, 2H), 1.70-1.50 (m, 3H), 1.30-1.20 (m, 4H), 0.80-0.70 (m, 2H)

**Example 5: Preparation of (3aS,4R,6aR)-1-((2R,3S)-2-amino-3-hydroxybutanoyl)-4-(4-boronobutyl)octahydropyrrolo[3,4-b]pyrrole-4-carboxylic acid dihydrochloride**

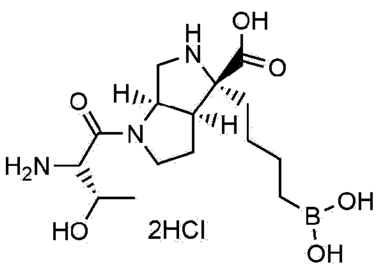

**5**

Compound 5 was obtained in the same manner as in steps 2 and 3 of Example 2, using Compound 2-1 (60 mg)

and 2,5-dioxopyrrolidin-1-yl (tert-butoxycarbonyl)-L-threoninate (40 mg).

MS: $[M+H]^+$= 358 $[M-H_2O+H]^+$= 340
$^1H$ NMR(500 MHz, MeOD) 3.94-3.40 (m, 1H), 3.50-3.40 (m, 3H), 3.40-3.30 (m, 1H), 3.10-2.85 (m, 2H), 2.05-1.80 (m, 3H), 1.60-1.50 (m, 2H), 1.40-1.20 (m, 4H), 1.20-1.15 (m, 3H), 0.9-0.8 (m, 2H)

**Example 6: Preparation of (3aS,4R,6aR)-1-((S)-2-amino-3-methylbutanoyl)-4-(4-boronobutyl)octahydropyrrolo [3,4-b]pyrrole-4-carboxylic acid dihydrochloride**

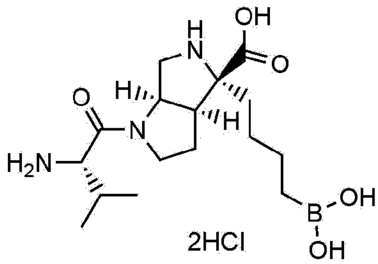

**6**

Compound 6 was obtained in the same manner as in steps 2 and 3 of Example 2, using Compound 2-1 (60 mg) and 2,5-dioxopyrrolidin-1-yl (tert-butoxycarbonyl)-L-valinate (40 mg).

MS: $[M+H]^+$= 356 $[M-H_2O+H]^+$= 338
$^1H$ NMR(500 MHz, MeOD) 4.74 (m, 1H), 4.1 (m, 1H), 4.05 (m, 1H), 3.81-3.80 (m, 1H), 3.56 (m, 2H), 3.22 (m, 1H), 2.24 (m, 2H), 2.09-2.07 (m, 2H), 1.96 (m, 1H), 1.52-1.44 (m, 3H), 1.30 (m, 1H), 1.09-1.04 (m, 6H), 0.81 (m, 2H)

**Example 7: Preparation of (3aS,4R,6aR)-1-((S)-2-amino-4-methylpentanoyl)-4-(4-boronobutyl)octahydropyrrolo[3,4-b]pyrrole-4-carboxylic acid dihydrochloride**

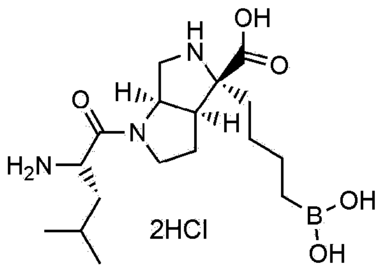

**7**

Compound 7 was obtained in the same manner as in steps 2 and 3 of Example 2, using Compound 2-1 (83 mg) and 2,5-dioxopyrrolidin-1-yl (tert-butoxycarbonyl)-L-leucinate (56 mg).

MS: $[M+H]^+$= 370,$[M-H_2O+H]^+$= 352
$^1H$ NMR(500 MHz, MeOD) 4.71 (m, 1H), 4.25 (m, 1H), 3.95 (m, 1H), 3.80 (m, 1H) 3.55 (m, 2H), 3.24 (m, 1H), 2.25 (m, 1H), 2.05-2.20 (m, 2H), 1.95 (m, 1H), 1.75 (m, 1H), 1.70 (m, 2H), 1.70-1.60 (m, 3H), 1.25 (m, 1H), 1.00-1.02 (m, 6H), 0.81-0.78 (m, 2H)

**Example 8: Preparation of (3aS,4R,6aR)-1-((2S,3S)-2-amino-3-methylpentanoyl)-4-(4-boronobutyl)octahydropyrrolo[3,4-b]pyrrole-4-carboxylic acid dihydrochloride**

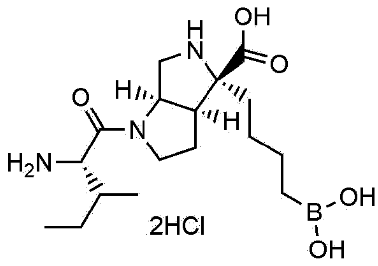

8

Compound 8 was obtained in the same manner as in steps 2 and 3 of Example 2, using Compound 2-1 (83 mg) and 2,5-dioxopyrrolidin-1-yl (tert-butoxycarbonyl)-L-isoleucinate (56 mg).

MS: $[M+H]^+$= 370, MS: $[M-H_2O+H]^+$= 352
$^1H$ NMR(500 MHz, MeOD) 4.72 (m, 1H), 4.09-3.95 (m, 2H), 3.76 (m, 1H), 3.57-3.54 (m, 2H), 2.20 (m, 1H), 2.10 (m, 2H), 1.95 (m, 2H), 2.70-2.40 (m, 4H), 2.20 (m, 1H), 2.15 (m, 2H), 1.07 (m, 3H), 0.96 (m, 3H), 0.81 (m, 2H)

**Example 9: Preparation of (3aS,4R,6aR)-1-((R)-2-aminopropanoyl)-4-(4-boronobutyl)octahydropyrrolo[3,4-b]pyrrole-4-carboxylic acid dihydrochloride**

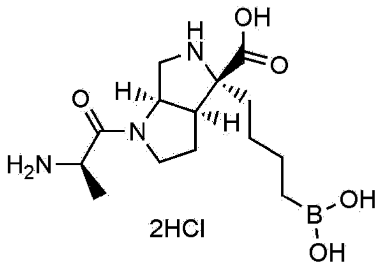

9

Compound 9 (15 mg) was obtained in the same manner as in steps 2 and 3 of Example 2, using Compound 2-1 (167 mg) and 2,5-dioxopyrrolidin-1-yl (tert-butoxycarbonyl)-D-alaninate (134 mg).

MS: $[M+H]^+$= 328
$^1H$ NMR(500 MHz, MeOD) 4.8(1H, m), 4.2(1H, m), 3.8(1H, m), 3.7(2H, m), 3.4(1H, m), 3.2(1H, m), 2.3(1H, m), 2.2(2H, m), 2.0-1.9(1H, m), 1.6-1.4(6H, m), 1.3(1H, m), 0.8(2H, m)

**Example 10: Preparation of 4-((3aS,4R,6aR)-4-(ethoxycarbonyl)octahydropyrrolo[2,3-c]pyrrol-4-yl)butylboronic acid dihydrochloride**

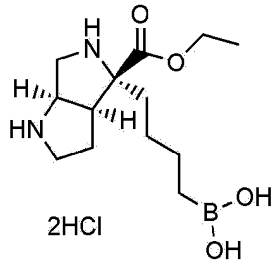

10

**Step 1) Preparation of (3aS,4R,6aR)-5-((benzoyloxy)carbonyl)-1-(tert-butoxycarbonyl)-4-(4-(4,4,5,5-tetramethyl-1,3,2-dioxaborolan-2-yl)butyl)octahydropyrrolo[3,4-b]pyrrole-4-carboxylic acid**

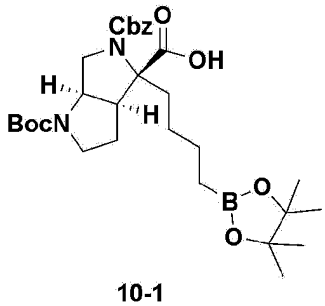

**10-1**

Compound 1-7 (1.0 g) was dissolved in methanol (20.0 ml). 10% Pd/C (100.0 mg) was added thereto, and stirred at room temperature for 4 hours using a hydrogen balloon to complete the reaction. The reaction solution was washed with methanol, filtered through Celite, and concentrated. It was dissolved in THF (20.0 ml), and then 1N-KOH (5.7 ml) was added , and benzyl carbonochloridate (0.43 ml) was slowly added dropwise, and then stirred at room temperature for 12 hours to complete the reaction. After the reaction solution was cooled to 0~5°C, ethyl acetate (10.0 ml) and $H_2O$ (5.0 ml) were added. After adjusting the pH to 2~3 using a 6N HCl aqueous solution, the layers were separated and the organic layer was separated. Ethyl acetate (10 ml) was added twice to the aqueous layer, the layers were separated and the organic layer was separated, and dried with $MgSO_4$, and then concentrated. The product was dried under reduced pressure to obtain Compound 9-1 (1.21 g, yield: 70%). The obtained compound was used for the next reaction without purification.
MS: $[M+H]^+$= 573

**Step 2) Preparation of 5-Benzyl 1-(tert-butyl) 4-ethyl (3aS,4R,6aR)-4-(4-(4,4,5,5-tetramethyl-1,3,2-dioxaborolan-2-yl)butyl)hexahydropyrrolo[3,4-b]pyrrole-1,4,5-tricarborate**

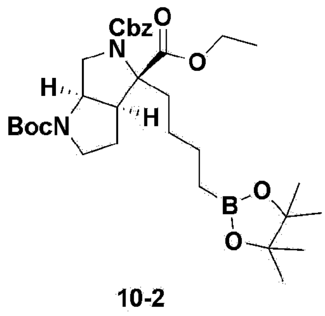

**10-2**

Compound 10-1 (100.0 mg) was dissolved in N,N-dimethylformamide (2.0 ml). Cesium carbonate (114.0 mg) and ethyl iodide (70.0 μL) were added and stirred at 50°C for 4 hours. After the reaction solution was cooled to 0-10°C, a saturated ammonium chloride aqueous solution (1.0 ml) and ethyl acetate (5.0 ml) were added and the organic layer was separated. The organic layer was dried over magnesium sulfate, concentrated, and subjected to a column separation using ethyl acetate:hexane=1:2 to obtain Compound 9-2 (80.0 mg. yield: 76%).
MS: $[M+H]^+$= 601

**Step 3) Preparation of 4-((3aS,4R,6aR)-4-(ethoxycarbonyl)octahydropyrrolo[2,3-c]pyrrol-4-yl)butylboronic acid dihydrochloride**

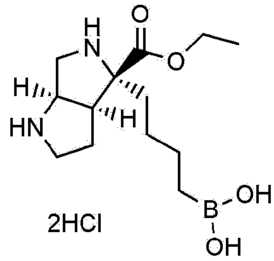

**10**

Compound 10(12.0 mg) was obtained in the same manner as in steps 8 to 10 of Example 1, using Compound 10-2.

MS: $[M+H]^+$= 285
$^1$H NMR(500 MHz, MeOD) : 4.7 (1H, m), 4.4(2H, m), 3.9(1H, m), 3.8(1H, m), 3.6(1H, m), 3.5(1H, m), 3.4(1H, m), 2.3(1H, m), 2.1(1H, m), 2.0(2H, m), 1.5(3H,m), 1.4(3H, m), 1.2(1H, m), 0.8(2H, m)

**Example 11: Preparation of 4-((3aS,4R,6aR)-4-(isopropoxycarbonyl)octahydropyrrolo[2,3-c]pyrrol-4-yl)butylboronic acid dihydrochloride**

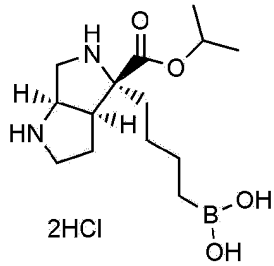

**11**

Compound 11 (17.7 mg) was obtained in the same manner as in steps 2 to 3 of Example 9, using Compound 9-1 (100.0 mg).

MS: $[M+H]^+$= 299
$^1$H NMR(500 MHz, MeOD) 5.2(1H, m), 4.8(1H, m),3.9-3.8(2H, m), 3.7-3.6(2H, m), 3.5(1H, m), 3.4(1H, m), 2.3(1H, m), 2.2-2.1(1H, m), 2.1-2.0(2H, m), 1.5(3H, m), 1.4(6H, m), 0.8(2H, m)

**Example 12: Preparation of 4-((3aS,4R,6aR)-4-(((isopropoxycarbonyloxy)methoxy)carbonyl)octahydropyrrolo[3,4-b]pyrrol-4-yl)butylboronic acid dihydrochloride**

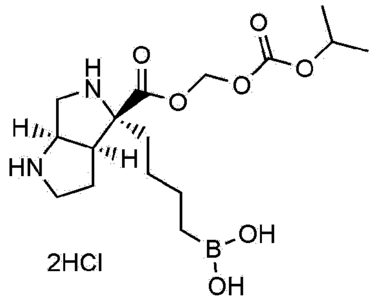

**12**

Compound 12 (40.0 mg) was obtained in the same manner as in Example 9, using Compound 9-1 (100.0 mg) and

chloromethyl isopropyl carbonate (35.0 μL).

MS: $[M+H]^+$= 373
$^1$H NMR(500 MHz, MeOD) : 6.0(2H, m), 4.7(1H, m), 3.9-3.8(2H, m), 3.5-3.4(3H,m), 3.4(1H, m), 2.4(1H, m), 2.1(3H,m), 1.5(3H, m), 1.25(1H,m), 1.2(6H, d), 0.8(2H, m)

**Example 13: Preparation of 4-((3aS,4R,6aR)-4-(((tert-butoxycarbonyloxy)methoxy)carbonyl)octahydropyrrolo [3,4-b]pyrrol-4-yl)butylboronic acid dihydrochloride**

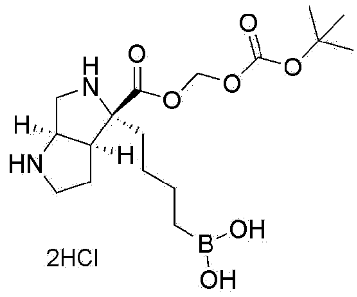

**13**

Compound 13 (10.0 mg) was obtained in the same manner as in Example 9, using Compound 9-1 (100.0 mg) and tert-butyl (chloromethyl) carbonate (37.8 μL).

MS: $[M+H]^+$= 387
$^1$H NMR(500 MHz, MeOD) 6.0(2H, m), 4.7(1H, m), 3.9-3.8(2H, m), 3.5-3.4(2H, m), 3.4(1H, m), 2.4-2.3(1H, m), 2.1-2.0(3H, m), 1.5-1.4(3H, m), 1.25(1H, m), 1.2(9H, s), 0.8(2H,m)

**Example 14: Preparation of 4-((3aS,4R,6aR)-4-((1-(ethoxycarbonyloxy)ethoxy)carbonyl)octahydropyrrolo[3,4-b]pyrrol-4-yl)butylboronic acid dihydrochloride**

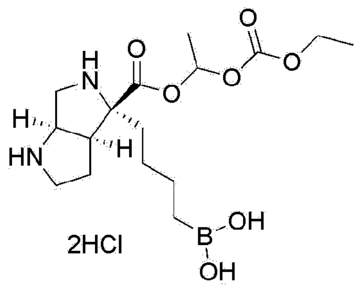

**14**

Compound 14 (15.0 mg) was obtained in the same manner as in Example 9, using Compound 9-1 (100.0 mg) and 1-chloroethyl ethyl carbonate(34.7 μL).

MS: $[M+H]^+$= 373
$^1$H NMR(500 MHz, MeOD) 6.9(1H, m), 4.7(1H, m), 3.9-3.8(2H, q), 3.7-3.5(2H, m), 3.5-3.4(3H, m), 2.3(1H, m), 2.2-2.1 (3H, m), 1.6(3H d), 1.6-1.5(4H, m), 1.4-1.3(6H, d), 0.8(2H, m)

**Example 15: Preparation of 4-((3aS,4R,6aR)-4-((1-(cyclohexyloxycarbonyloxy)ethoxy)carbonyl)octahydropyrrolo[3,4-b]pyrrol-4-yl)butylboronic acid dihydrochloride**

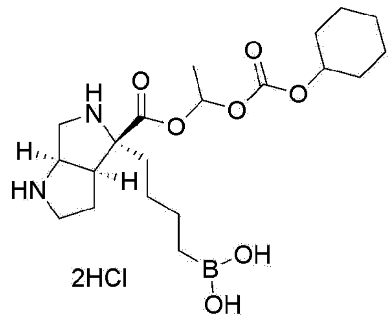

**15**

Compound 15 (12.0 mg) was obtained in the same manner as in Example 9, using Compound 9-1 (100.0 mg) and 1-chloroethyl cyclohexyl carbonate(48.0 μL).

MS: $[M+H]^+$= 427
$^1$H NMR(500 MHz, MeOD) 6.9(1H, m), 4.7(1H, m), 3.9-3.8(2H, m), 3.5(2H, m), 3.4(1H, m), 2.3(1H, m), 2.2-2.0(3H, m), 1.9(2H, m), 1.7-1.6(2H, m), 1.5(3H, m), 1.5-1.3(11H, m), 0.8(2H, m)

**Example 16: Preparation of 4-((3aS,4R,6aR)-4-((1-(isopropoxycarbonyloxy)ethoxy)carbonyl)octahydropyrrolo [3,4-b]pyrrol-4-yl)butylboronic acid dihydrochloride**

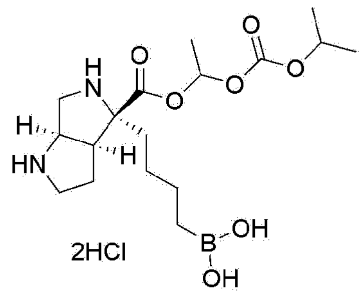

**16**

Compound 16 (15.0 mg) was obtained in the same manner as in Example 9, using Compound 9-1 (150.0 mg) and 1-chloroethyl cyclopropyl carbonate (60.0 μL).

MS: $[M+H]^+$= 387
$^1$H NMR(500 MHz, MeOD) 6.9(1H, m), 4.7(1H, m), 3.9-3.8(2H, m), 3.7-3.5(2H,m), 3.5-3.4(2H, m), 2.4-2.3(1H, m), 2.2-2.0(3H, m), 1.6(3H, m), 1.6-1.5(4H, m), 1.4-1.3(6H, d), 0.8(2H, m)

**Example 17: Preparation of 4-((3aS,4R,6aR)-4-((1-(pivaloyloxy)ethoxy)carbonyl)octahydropyrrolo[3,4-b]pyrrol-4-yl)butylboronic acid dihydrochloride**

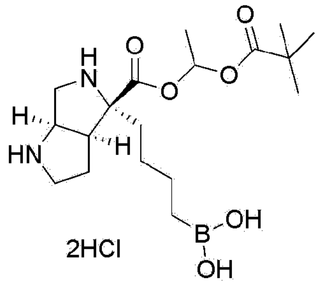

**17**

Compound 17 (20.0 mg) was obtained in the same manner as in Example 9, using Compound 9-1 (100.0 mg) and 1-chloroethyl 1-chloroethyl pivalate (47.0 μL).

MS: $[M+H]^+$= 385
$^1$H NMR(500 MHz, MeOD) 7.0(1H, m), 4.8(1H, m), 4.0-3.8(2H, m), 3.6-3.5(2H, m), 3.4(1H, m), 2.3-2.1(1H, m), 2.2-2.0(4H, m), 1.6(3H, m), 1.5(3H, m), 1.3-1.2(9H, s), 0.8(2H, m)

**Example 18: Preparation of 4-((3aS,4R,6aR)-4-((2-(pivaloyloxy)ethoxy)carbonyl)octahydropyrrolo[3,4-b]pyrrol-4-yl)butylboronic acid dihydrochloride**

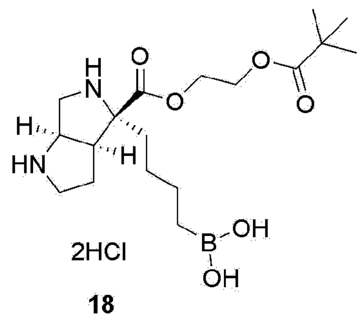

**18**

Step 1) Preparation of 5-Benzyl 1-(tert-butyl) 4-(2-hydroxyethyl) (3aS,4R,6aR)-4-(4-(4,4,5,5-tetramethyl-1,3,2-dioxaborolan-2-**yl)butyl)hexahydroprolo[3,4-b]pyrrole-1,4,5-tricarboxylate**

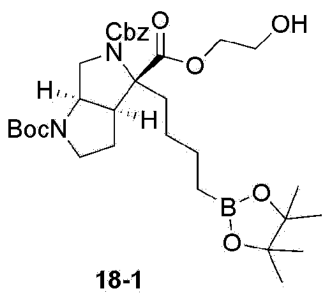

**18-1**

Compound 18-1 (197.0 mg, yield: 91%) was obtained in the same manner as in Example 9, using Compound 9-1 (200.0 mg) and 2-bromoethane-1-ol (125.0 μL).
MS: $[M+H]^+$= 617

**Step 2) Preparation of 5-Benzyl 1-(tert-butyl) 4-(2-(pivaloyloxy)ethyl) (3aS,4R,6aR)-4-(4-(4,4,5,5-tetramethyl-1,3,2-dioxaborolan-2-yl)butyl)hexahydroprolo[3,4-b]pyrrole-1,4,5-tricarboxylate**

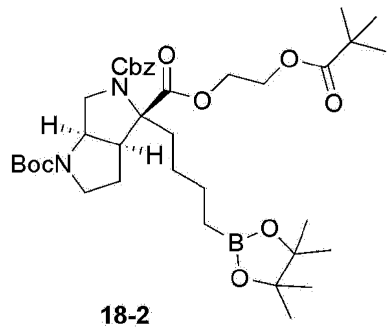

**18-2**

Compound 18-1 (200.0 mg) was dissolved in tetrahydrofuran (3.0 ml), and then triethylamine (136.0 μL) and pivaloyl chloride (90.8 μL) were added thereto. The mixture was reacted at room temperature for 12 hours, then concentrated and subjected to a column separation with ethyl acetate: hexane = 1:1 to obtain Compound 18-2 (60.0 mg). MS: $[M+H]^+$= 701

**Step 3) Preparation of 4-((3aS,4R,6aR)-4-((2-(pivaloyloxy)ethoxy)carbonyl)octahydropyrrolo[3,4-b]pyrrol-4-yl) butylboronic acid dihydrochloride**

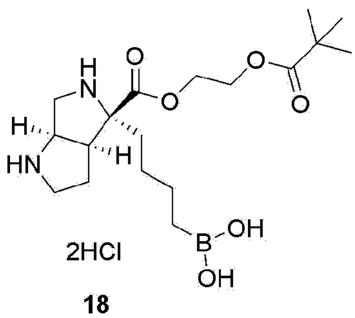

2HCl

**18**

Compound 18 (14.0 mg) was obtained in the same manner as in steps 8 to 10 of Example 1, using Compound 18-2 (60.0 mg).

MS: $[M+H]^+$= 385
$^1H$ NMR(500 MHz, MeOD) 4.7(1H, m), 4.6-4.5(2H, m), 4.4(2H, m), 4.0-3.8(3H, m), 3.8-3.6(2H, m), 2.4(1H, m), 2.1-2.0(3H, m), 1.4-1.4(3H, m), 1.3(1H, m), 1.3-1.2(9H, m), 0.8(2H, m)

**Example 19: Preparation of (3aS,4R,6aR)-4-(4-boronobutyl)-1-((1-(cyclohexanecarbonyloxy)ethoxy)carbonyl) octahydropyrrolo[3,4-b]pyrrole-4-carboxylic acid hydrochloride**

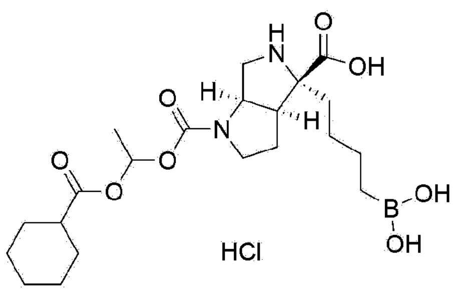

HCl

**19**

**Step 1) Preparation of 4,5-Dibenzyl 1-(1-chloroethyl)(3aS,4R,6aR)-4-(4-(4,4,5,5-tetramethyl-1,3,2-dioxaborolan-2-yl)butyl)hexahydropyrrolo[3,4-b]pyrrole-1,4,5-tricarboxylate**

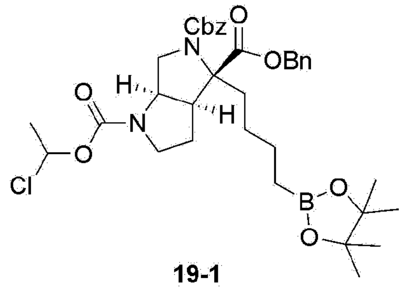

**19-1**

Compound 2-1 (431.1 mg) was dissolved in dimethyl chloride (5.0 ml). The solution was cooled to 0-10°C, and then triethylamine (300.0 μL) was added. 1-Chloroethyl carbon chloride (118.0 μL) was slowly added dropwise, and then stirred at room temperature for 12 hours to complete the reaction. After the reaction solution was cooled to 0~10°C, a saturated ammonium chloride aqueous solution was added, and the organic layer was separated. The organic layer was dried over $MgSO_4$, concentrated and dried under reduced pressure to obtain Compound 19-1 (455.0 mg).
MS: $[M+H]^+$= 670

**Step 2) Preparation of (3aS,4R,6aR)-4-(4-boronobutyl)-1-((1-(cyclohexanecarbonyloxy)ethoxy)carbonyl)octahydropyrrolo[3,4-b]pyrrole-4-carboxylic acid hydrochloride**

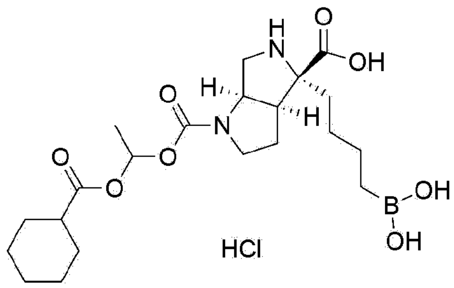

**19**

Compound 19-1 (235.4 mg) was dissolved in dimethyl chloride (3.0 ml). Cesium carbonate (234.0 mg), sodium iodide (5.4 mg), and cyclohexanecarboxylic acid (67.0 μL) were added. The mixture was stirred at 50°C for 2 hours to complete the reaction. After the reaction solution was cooled to 0-10°C, ethyl acetate (5.0 ml) and a saturated ammonium chloride aqueous solution (5.0 ml) were added, the layers were separated, and the organic layer was concentrated. The product was subjected to a column separation with ethyl acetate:hexane=3:7, and Compound 19 (20.0 mg) was obtained in the same manner as in steps 8 to 10 of Example 1.

MS: $[M+H]^+$= 455
$^1H$ NMR(500 MHz, MeOD) 6.7(1H, m), 4.8-4.7(1H,m), 3.8-3.7(1H, m), 3.6-3.5(1H, m), 3.4(2H, m), 3.2(1H, m), 2.3(1H, m), 2.2(1H, m), 2.1(1H,m), 1.9-1.8(4H, m), 1.7(2H, m), 1.6(1H, m), 1.4(8H, m), 1.3(4H, m), 0.8(2H, m)

**Example 20: Preparation of (3aS,4R,6aR)-4-(4-boronobutyl)-1-((1-(pentanoyloxy)ethoxy)carbonyl)octahydropyrrolo[3,4-b]pyrrole-4-carboxylic acid hydrochloride**

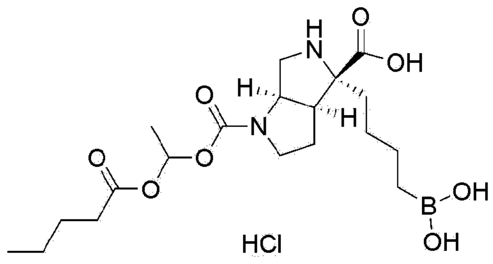

**20**

Compound 20 (7.0 mg) was obtained in the same manner as in Example 19, using Compound 19-1 (100.0 mg) and pentanoic acid (35.0 μL).

MS: $[M+H]^+$= 429
$^1$H NMR(500 MHz, MeOD) 6.5(1H, m), 4.5-4.4(1H, m), 3.6(2H, m), 3.5-3.4(2H, m), 3.0(1H, m), 2.4(2H, m), 2.2(1H, m), 2.1-2.0(2H, m), 1.9-1.8(1H, m), 1.7(1H, m), 1.6(2H,m), 1.5-1.3(5H, m), 1.0-0.9(9H, m), 0.8-0.6(2H, m)

**Example 21: Preparation of (3aS,4R,6aR)-4-(4-boronobutyl)-1-((1-(pivaloyloxy)ethoxy)carbonyl)octahydropyrrolo[2,3-c]pyrrole-4-carboxylic acid hydrochloride**

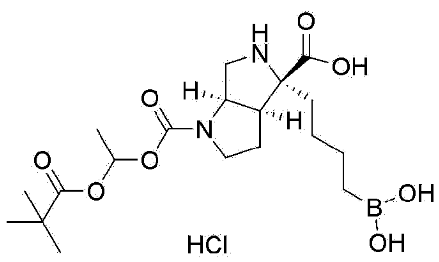

**21**

Compound 21 (15.0 mg) was obtained in the same manner as in Example 19, using Compound 19-1 (200.0 mg) and pivalic acid (70.0 μL) .

MS: $[M+H]^+$= 429
$^1$H NMR(500 MHz, MeOD) 6.7(1H, m), 4.7(1H, m), 3.8-3.7(1H, m), 3.6-3.5(1H, m), 3.5-3.4(2H, m), 3.2(1H, m), 2.2(1H, m), 2.1(1H, m), 2.0(2H,m), 1.5-1.4(6H, m), 1.3-1.2(1H,m), 1.2(9H, m), 0.8(2H, m)

**Example 22: Preparation of (3aS,4R,6aR)-4-(4-boronobutyl)-1-((1-((S)-2-(dimethylamino)-3-methylbutanoyloxy) ethoxy)carbonyl)octahydropyrrolo[3,4-b]pyrrole-4-carboxylic acid hydrochloride**

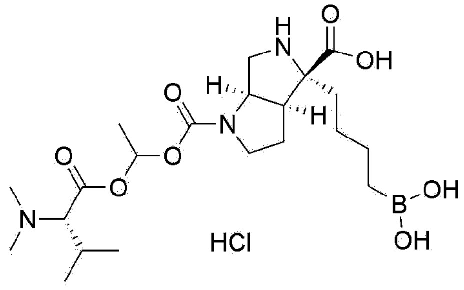

**22**

Compound 22 (15.5 mg) was obtained in the same manner as in Example 19, using Compound 19-1 (240 mg) and dimethyl-L-valine (78.4 mg).

MS: $[M+H]^+$= 472
$^1$H NMR(500 MHz, MeOD) 6.9(1H,m), 4.8-4.7(1H, m), 4.1-4.0(1H, m), 3.9-3.5(2H, m), 3.5-3.4(2H, m), 3.2-3.1(1H, m), 2.9(6H, s), 2.5(1H, m), 2.2-1.8(3H, m), 1.7-1.5(3H, m), 1.5(2H, m), 1.4(2H, m), 1.2(3H, m), 1.1(3H, m), 0.8(2H, m)

**Example 23: Preparation of (3aS,4R,6aR)-4-(4-boronobutyl)-1-((1-(cyclohexanecarbonyloxy)-2-methylpropoxy) carbonyl)octahydropyrrolo[3,4-b]pyrrole-4-carboxylic acid hydrochloride**

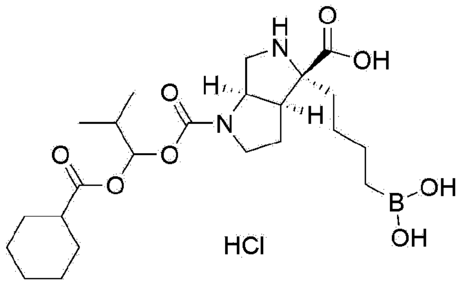

**23**

**Step 1) Preparation of 4,5-Dibenzyl 1-(1-chloro-2-methylpropyl)(3aS,4R,6aR)-4-(4-(4,4,5,5-tetramethyl-1,3,2-dioxaborolan-2-yl)butyl)hexahydroprolo[3,4-b]pyrrole-1,4,5-tricarboxylate**

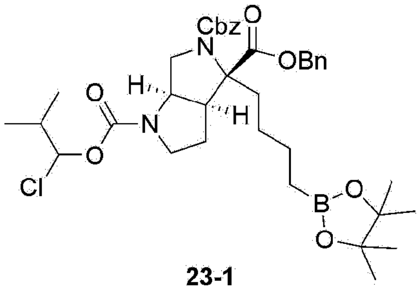

**23-1**

Compound 2-1 (300 mg) was dissolved in dimethyl chloride (3.0 ml). The solution was cooled to 0~10°C, and then triethylamine(200.0 μL) was added. 1-Chloro-2-methylpropyl carbon chloride (125.0 μL) was slowly added dropwise, and then stirred at room temperature for 12 hours to complete the reaction. After the reaction solution was cooled to 0-10°C, a saturated ammonium chloride aqueous solution was added, and the organic layer was separated. The organic layer was dried over $MgSO_4$, concentrated and dried under reduced pressure to obtain Compound 23-1 (350.0 mg).
MS: $[M+H]^+$= 698

**Step 2) Preparation of (3aS,4R,6aR)-4-(4-boronobutyl)-1-((1-(cyclohexanecarbonyloxy)-2-methylpropoxy)carbonyl)octahydropyrrolo[3,4-b]pyrrole-4-carboxylic acid hydrochloride**

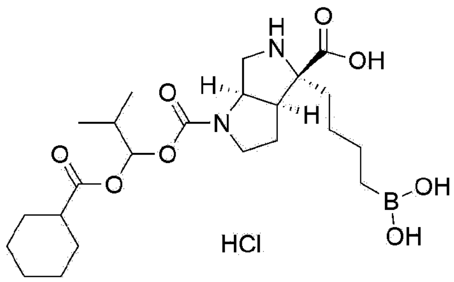

**23**

Compound 23-1 (115.0 mg) was dissolved in dimethylformamide (1.0 ml). Cesium carbonate (108.0 mg), sodium iodide (3.0 mg), and cyclohexanecarboxylic acid (32.0 μL) were added thereto. The mixture was stirred at 70°C for 2 hours to complete the reaction. After the reaction solution was cooled to 0-10°C, ethyl acetate (5.0 ml) and saturated ammonium chloride aqueous solution (3.0 ml) were added, the layers were separated, and the organic layer was concentrated. The product was subjected to a column separation with ethyl acetate:hexane=3:7, and Compound 23 (20.0 mg) was obtained in the same manner as in steps 8 to 10 of Example 1.

MS: $[M+H]^+$= 483
$^1H$ NMR(500 MHz, MeOD)6.8(1H, m), 4.8-4.5(1H, m), 3.6(2H,m), 3.4(2H,m), 3.1-2.9(2H, m) 2.3(2H, m), 2.1-2.0(2H,m), 1.9(3H, m), 2.8-2.6(4H, m), 1.5-1.3(4H, m), 1.3-1.2(3H, m), 1.0(6H, m), 0.9-0.8(2H, m)

**Example 24: Preparation of (3aS,4R,6aR)-4-(4-boronobutyl)-1-((2-methyl-1-(pivaloyloxy)propoxy)carbonyl)octahydropyrrolo[3,4-b]pyrrole-4-carboxylic acid hydrochloride**

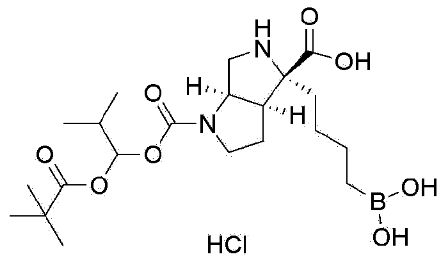

**24**

Compound 24 (5.6 mg) was obtained in the same manner as in Example 23, using Compound 23-1 (115 mg) and pivalic acid (25.6 mg).

MS: $[M+H]^+$= 457
$^1H$ NMR(500 MHz, MeOD) 6.5(1H, m), 4.5-4.4(1H, m), 3.6(1H, m), 3.5-3.4(2H, m), 3.1-2.9(2H, m), 2.2-2.0(2H, m), 1.9-1.8(1H, m), 1.7(2H, m), 1.5-1.4(3H, m), 1.2(9H, m), 1.0(6H, m), 0.8-0.6(2H,m)

**Example 25: Preparation of (3aS,4R,6aR)-4-(4-boronobutyl)-1-(ethoxycarbonyl)octahydropyrrolo[3,4-b]pyrrole-4-carboxylic acid hydrochloride**

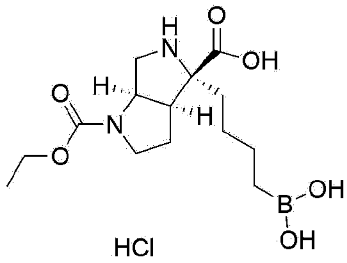

**25**

Compound 25 (14 mg) was obtained in the same manner as in step 1 of Example 19 and steps 8 to 10 of Example 1, using Compound 2-1 (50 mg) and ethyl carbonochloridate (12 μL).

MS: $[M+H]^+$= 329
$^1$H NMR(500 MHz, MeOD) 4.6(1H, m), 4.1(2H, m), 3.7(1H, m), 3.6(1H, m),3.5-3.4(2H, m), 3.2(1H, m), 2.2(1H, m), 2.1(1H, m), 1.9(2H, m), 1.5(3H, m), 1.3(4H, m), 0.8(2H, m)

**Example 26: Preparation of (3aS,4R,6aR)-4-(4-boronobutyl)-1-(isobutoxycarbonyl)octahydropyrrolo[3,4-b]pyrrole-4-carboxylic acid hydrochloride**

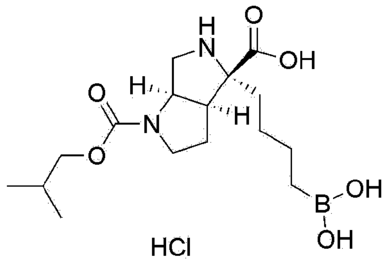

**26**

Compound 26 (6.7 mg) was obtained in the same manner as in step 1 of Example 19 and steps 8 to 10 of Example 1, using Compound 2-1 (50 mg) and isobutyl carbonochloridate (15 μL).

MS: $[M+H]^+$= 357
$^1$H NMR(500 MHz, MeOD) 4.5(1H, m), 3.9(2H, m), 3.7-3.5(2H, m), 3.4(2H, m), 3.2(1H, m), 2.2(1H, m), 2.1(1H, m), 2.0-1.9(3H, m), 1.6-1.5(3H, m), 1.3(1H, m), 1.0(6H, m), 0.8(2H, m)

**Example 27: Preparation of (3aS,4R,6aR)-4-(4-boronobutyl)-1-methyloctahydropyrrolo[3,4-b]pyrrole-4-carboxylic acid hydrochloride**

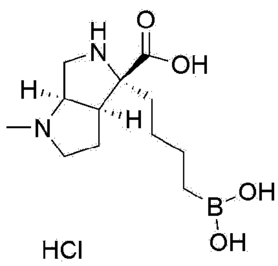

**27**

After Compound 2-1 (100 mg) was dissolved in dichloromethane (2 ml), formaldehyde (12.3 μL) and acetic acid (40.0 μL) were added, and then stirred at 40°C for 3 hours. Sodium acetoxyborohydride (96 mg) was added thereto, and then stirred at 40°C for 3 hours to complete the reaction. After the reaction solution was cooled to 0-10°C, $H_2O$ was added to separate the organic layer. The product was concentrated, and separated with ethyl acetate:hexane= 9:1, and then Compound 27 (4.0 mg) was obtained in the same manner as in steps 8 to 10 of Example 1.

MS: $[M+H]^+$= 271
$^1H$ NMR(500 MHz, MeOD) 4.5(1H, m), 4.2(1H, m), 3.9(1H, m), 3.8(1H, m), 3.7-3.5(2H, m), 3.1(3H, s), 2.5(1H, m), 2.2-2.1(2H, m), 2.0(1H, m), 1.6(1H, m), 1.5(2H, m), 1.3(1H, m), 0.8(2H, m)

**Example 28: Preparation of (3aS,4R,6aR)-4-(4-boronobutyl)-1-(2-(methylamino)ethyl)octahydropyrrolo[2,3-c]pyrrole-4-carboxylic acid hydrochloride**

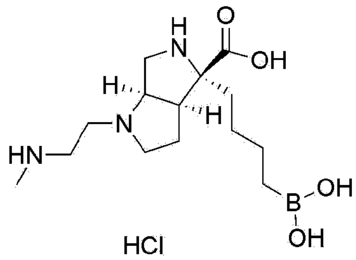

**28**

After Compound 2-1 (200 mg) was dissolved in dichloromethane (2 ml), tert-butyl methyl(2-oxoethyl)carbamate (115.6 μL) and acetic acid (95 μL) were added, and then stirred at 40°C for 3 hours. Sodium acetoxyborohydride (200 mg) was added thereto, and then stirred at 40°C for 3 hours to complete the reaction. After the reaction solution was cooled to 0-10°C, $H_2O$ was added, and the organic layer was separated. The product was concentrated, and separated with ethyl acetate: hexane=5:1, and then Compound 28 (20.0 mg) was obtained in the same manner as in steps 8 to 10 of Example 1.

MS: $[M+H]^+$= 314
$^1H$ NMR(500 MHz, $D_2O$) 4.3(1H, m), 3.8(1H, m), 3.7(1H, m), 3.6-3.4(3H, m), 3.4-3.3(2H, m), 3.2(1H, m), 3.1(1H, m), 2.3(1H, m), 2.0(1H, m), 1.8(2H, m), 1.5-1.3(2H, m), 1.1(1H, m), 0.7-0.6(2H, m)

**Experimental Example 1: Arginase Inhibitory Activity Test**

An arginase inhibitory activity test was conducted for the compound according to the present disclosure. In addition to the compound according to the present disclosure, Compound CB - 1158 (Numidargistat) was also tested together for comparison.

Specifically, all reagents required for evaluation were diluted in a reaction buffer (8 mM $NAHPO_4$, 2 mM $KH_2PO_4$, pH 7.5, 137 mM NaCl, 2.7 mM KCl, and 0.05% Tween-20), and the compound was dissolved in DMSO and then diluted, and then diluted in the reaction buffer to conform to the desired concentration. 10 μL of the compound and 10 μL of 30 nM Arginase-1 were mixed in a transparent 96-well plate (SPL), and incubated at room temperature for 1 hour or more. After that, 10 μL of 15 mM L-arginine and 3 mM $MnCl_2$ were added to the plate, and then reacted at room temperature for 30 minutes or more. Finally, 30 μL of a 1:1 mixture of reagent A (10 mM o-phthaldialdehyde, 0.4% polyoxyethylene lauryl ether, and 1.8M sulfuric acid in DW) and reagent B (1.3 mM primaquine diphosphate, 0.4% polyoxyethylene lauryl ether, and 3.6 M sulfuric acid in DW) was added to complete the enzyme reaction, and incubated at room temperature for 1 hour or more. The plate after incubation was measured for absorbance at 450 nM using a Flexstation3 multi-mode microplate reader (Molecular Devices).

The background control(BC) reading value was subtracted from all reading values to obtain the ΔOD for each reading value. The ΔOD of the enzyme control (EC) was set to the maximum value, and the % relative Arginase-1 activity of each compound (S) was calculated using the following Equation, and the $IC_{50}$ value of the compound was determined using PRISM (GraphPad software).

$$\text{Relative Arginase-1 activity (\%)} = (\Delta\text{OD of S}/\Delta\text{OD of EC})*100$$

Table 1 below lists the efficacies of the compounds on a scale from A to C. The efficacy value of A refers to a compound of the present disclosure having an $IC_{50}$ value of less than 100 nM, a compound having an efficacy value of B exhibits an $IC_{50}$ value in the range of 100 nM to 1,000 nM, and a compound having an $IC_{50}$ value of greater than 1,000 nM was assigned an efficacy value of C.

[Table 1]

| Compound | $IC_{50}$ |
|---|---|
| CB-1158 (Numidargistat) | A |
| Example 1 | A |
| Example 2 | B |
| Example 3 | B |
| Example 4 | B |
| Example 5 | B |
| Example 6 | C |
| Example 7 | C |
| Example 9 | C |
| Example 27 | A |
| Example 28 | B |

**Experimental Example 2: PBMC Activity Evaluation Test**

A Primary Peripheral Blood Mononuclear Cells (PBMC) activity test was performed for the compound according to the present disclosure. It was confirmed that T cell differentiation induced using α-CD3 and α-CD28 antibodies was inhibited via synthetic arginase1, and then the compound inhibited arginase1 and induced T cell differentiation and proliferation. To evaluate the proliferation of T cells, the CellTiter-Glo® Luminescent Cell Viability (Promega, Catalog #G7571) evaluation method was used, which can confirm the proliferation of cells by quantifying the ATP present in the cells. In addition to the compound according to the present disclosure, the compound CB-1158 (Numidargistat) was also tested together for comparison.

Specifically, α-CD3 antibody was coated on a clear 96-well microplate. $1\times10^5$ PBMC cells were placed in each well. RPMI media (10% FBS, 1% penicillin/streptomycin) was used as the cell culture medium. α-CD28 antibody and synthetic arginase 1 were added to each well. The compounds prepared in Examples were treated at different concentrations (2, 1, 0.5, 0.25, 0.125, 0.0625, 0.03125 μM). After incubating for 72 hours, 50 μL of cells from each well were transferred to a white 96-well microplate, and mixed with 50 μL of CellTiter-Glo® solution.

After performing the reaction at room temperature for 10 minutes in the state where light was blocked, the luminescence was measured. The measurement results for each compound were calculated using Excel, and the $EC_{50}$ value was calculated using GraphPad Prism software.

Table 2 below lists the efficacies of the compounds on a scale of A and B. The efficacy value of A refers to a compound of the present disclosure having an $EC_{50}$ value of less than 100 nM, and the compound having an $EC_{50}$ value in the range of 100 nM to 500 nM was assigned an efficacy value of B.

[Table 2]

| In vitro activity | CB-1158 | Example 1 |
|---|---|---|
| $EC_{50}$ | B | A |

## Claims

1. A compound represented by the following Chemical Formula 1, or a pharmaceutically acceptable salt thereof:

[Chemical Formula 1]

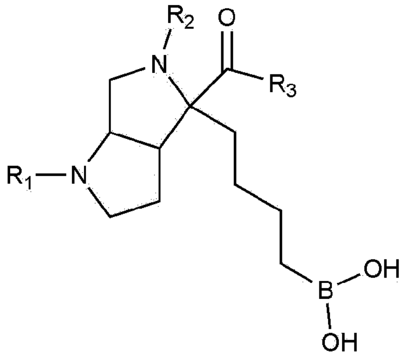

in Chemical Formula 1,

$R_1$ and $R_2$ are each independently hydrogen; a $C_{1-4}$ alkyl which is unsubstituted or substituted with amino, ($C_{1-4}$ alkyl)amino, or di($C_{1-4}$ alkyl)amino; or - $L_1$-$R'_1$,

$L_1$ is -CO-, -CO-O-, -(CO)-O-($C_{1-4}$ alkylene)-O-(CO)-, or -$CH_2$-O-CO-,
$R'_1$ is -CH($NH_2$)-$R_A$, -CH(N($CH_3$)$_2$)-$R_A$, $R_B$, or Rc,

$R_3$ is hydroxy, or -$L_3$-$R'_3$,

$L_3$ is -NH-, -O-, or -O-($CH_2$)$_{n3}$-O-CO-,
$R'_3$ is -CH(COOH)-$R_A$, -CH($NH_2$)-$R_A$, $R_B$, or Rc,

each $R_A$ is independently hydrogen; or a $C_{1-6}$ alkyl which is unsubstituted or substituted with hydroxy, mercapto, hydroseleno, guanidino, amino, carboxy, carbamoyl, $C_{1-6}$ alkylthio, phenyl, hydroxyphenyl, or $C_{4-10}$ heteroaryl containing one or two N,
each $R_B$ is independently a $C_{4-10}$ heterocycloalkyl containing N,
each Rc is independently a $C_{1-6}$ alkyl group which is unsubstituted or substituted with a $C_{6-10}$ aryl or a $C_{4-10}$ heterocycloalkyl containing N; a $C_{6-10}$ aryl which is unsubstituted or substituted with a $C_{1-6}$ alkoxy; or a $C_{3-6}$ cycloalkyl, and
n3 is an integer of 1 to 3.

**2.** The compound or the pharmaceutically acceptable salt thereof according to claim 1, wherein:

$R_1$ and $R_2$ are each independently hydrogen, methyl, methylaminoethyl, or a substituent represented by any one of the following:

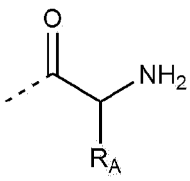
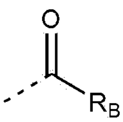
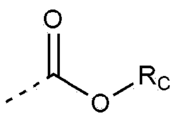
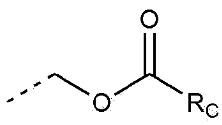

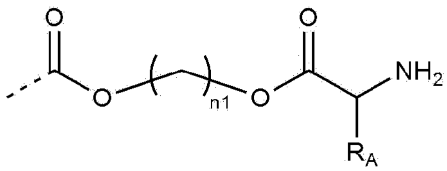
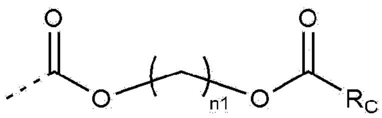

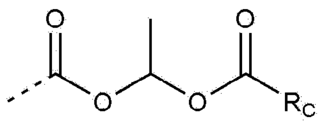 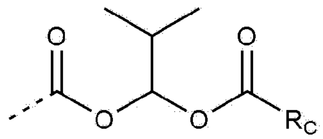

wherein,
$R_A$, $R_B$, and Rc are as defined in claim 1, and
n1 is an integer of 1 to 3.

**3.** The compound or the pharmaceutically acceptable salt thereof according to claim 1, wherein:

$R_3$ is hydroxy, or a substituent represented by any one of the following:

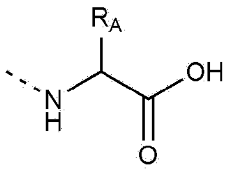 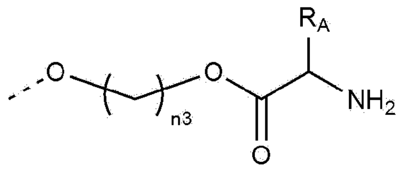

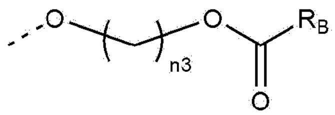

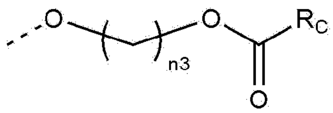 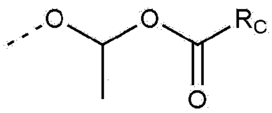 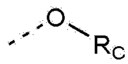

wherein,
$R_A$, $R_B$, Rc and n3 are as defined in claim 1.

**4.** The compound or the pharmaceutically acceptable salt thereof according to claim 1, wherein: each $R_A$ is independently hydrogen; or a $C_{1-6}$ alkyl which is unsubstituted or substituted with hydroxy, mercapto, hydroseleno, guanidino, amino, carboxy, carbamoyl, methylthio, phenyl, hydroxyphenyl, indole, or imidazole.

**5.** The compound or the pharmaceutically acceptable salt thereof according to claim 1, wherein:

each $R_A$ is independently a residue of a natural amino acid,
wherein the residue of the natural amino acid means a structure excluding the terminal $-CH(NH_2)(COOH)$.

**6.** The compound or the pharmaceutically acceptable salt thereof according to claim 1, wherein: Rs is pyrrolidinyl,

**7.** The compound or the pharmaceutically acceptable salt thereof according to claim 1, wherein: each Rc is independently a $C_{1-6}$ alkyl which is unsubstituted or substituted with phenyl, or piperidinyl; a $C_{6-10}$ aryl which is unsubstituted or substituted with methoxy; or a $C_{3-6}$ cycloalkyl.

**8.** The compound or the pharmaceutically acceptable salt thereof according to claim 1, wherein:

$R_1$ is hydrogen, and $R_2$ is hydrogen, or
$R_1$ is hydrogen, and $R_3$ is hydroxy, or
$R_2$ is hydrogen, and $R_3$ is hydroxy.

**9.** The compound or the pharmaceutically acceptable salt thereof according to claim 1, wherein:

the Chemical Formula 1 is represented by any one of the following Chemical Formulas 2 to 5:

[Chemical Formula 2]

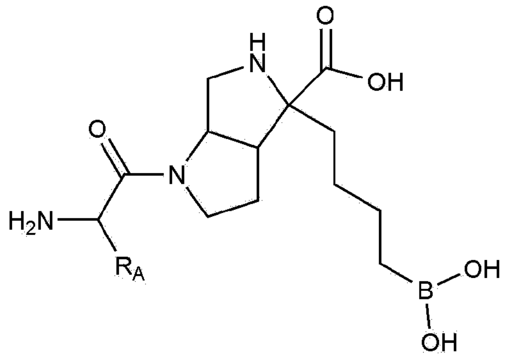

[Chemical Formula 3]

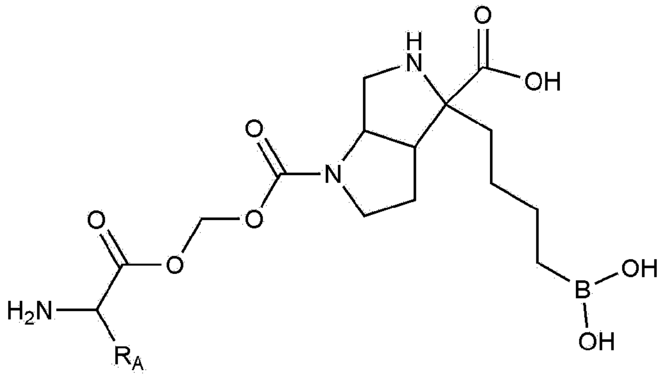

[Chemical Formula 4]

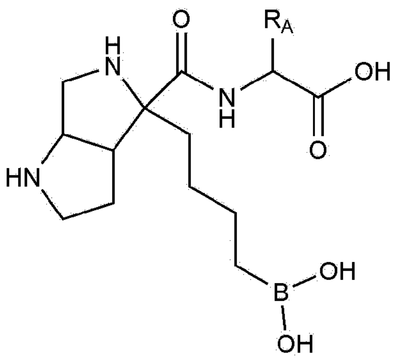

[Chemical Formula 5]

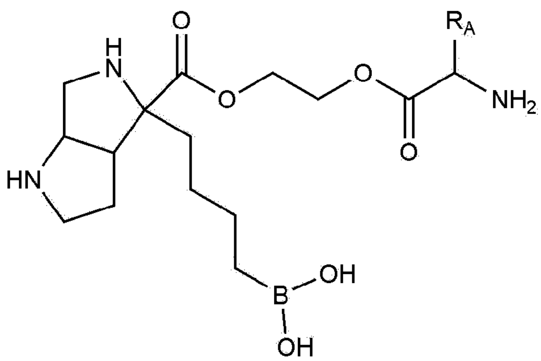

wherein in Chemical Formulas 2 to 5,
$R_A$ is as defined in claim 1.

**10.** The compound or the pharmaceutically acceptable salt thereof according to claim 1, wherein the compound represented by Chemical Formula 1 is any one selected from the group consisting of:

1) (3aS,4R,6aR)-4-(4-boronobutyl)octahydropyrrolo[2,3-c]pyrrole-4-carboxylic acid,
2) (3aS,4R,6aR)-1-((S)-2-aminopropanoyl)-4-(4-boronobutyl)octahydropyrrolo[3,4-b]pyrrole-4-carboxylic acid,
3) (3aS,4R,6aR)-1-((S)-2-amino-3-phenylpropanoyl)-4-(4-boronobutyl)octahydropyrrolo[3,4-b]pyrrole-4-carboxylic acid,
4) (3aS,4R,6aR)-4-(4-boronobutyl)-1-((S)-pyrrolidine-2-carbonyl)octahydropyrrolo[3,4-b]pyrrole-4-carboxylic acid,
5) (3aS,4R,6aR)-1-((2R,3S)-2-amino-3-hydroxybutanoyl)-4-(4-boronobutyl)octahydropyrrolo[3,4-b]pyrrole-4-carboxylic acid,
6) (3aS,4R,6aR)-1-((S)-2-amino-3-methylbutanoyl)-4-(4-boronobutyl)octahydropyrrolo[3,4-b]pyrrole-4-carboxylic acid,
7) (3aS,4R,6aR)-1-((S)-2-amino-4-methylpentanoyl)-4-(4-boronobutyl)octahydropyrrolo[3,4-b]pyrrole-4-carboxylic acid,
8) (3aS,4R,6aR)-1-((2S,3S)-2-amino-3-methylpentanoyl)-4-(4-boronobutyl)octahydropyrrolo[3,4-b]pyrrole-4-carboxylic acid,
9) (3aS,4R,6aR)-1-((R)-2-aminopropanoyl)-4-(4-boronobutyl)octahydropyrrolo[3,4-b]pyrrole-4-carboxylic acid,
10) 4-((3aS,4R,6aR)-4-(ethoxycarbonyl)octahydropyrrolo[2,3-c]pyrrol-4-yl)butylboronic acid,
11) 4-((3aS,4R,6aR)-4-(isopropoxycarbonyl)octahydropyrrolo[2,3-c]pyrrol-4-yl)butylboronic acid,
12) 4-((3aS,4R,6aR)-4-(((isopropoxycarbonyloxy)methoxy)carbonyl)octahydropyrrolo[3,4-b]pyrrol-4-yl)butylboronic acid,
13) 4-((3aS,4R,6aR)-4-(((tert-butoxycarbonyloxy)methoxy)carbonyl)octahydropyrrolo[3,4-b]pyrrol-4-yl)butylboronic acid,
14) 4-((3aS,4R,6aR)-4-((1-(ethoxycarbonyloxy)ethoxy)carbonyl)octahydropyrrolo[3,4-b]pyrrol-4-yl)butylboronic acid,
15) 4-((3aS,4R,6aR)-4-((1-(cyclohexyloxycarbonyloxy)ethoxy)carbonyl)octahydropyrrolo[3,4-b]pyrrol-4-yl)butylboronic acid,
16) 4-((3aS,4R,6aR)-4-((1-(isopropoxycarbonyloxy)ethoxy)carbonyl)octahydropyrrolo[3,4-b]pyrrol-4-yl)butylboronic acid,
17) 4-((3aS,4R,6aR)-4-((1-(pivaloyloxy)ethoxy)carbonyl)octahydropyrrolo[3,4-b]pyrrol-4-yl)butylboronic acid,
18) 4-((3aS,4R,6aR)-4-((2-(pivaloyloxy)ethoxy)carbonyl)octahydropyrrolo[3,4-b]pyrrol-4-yl)butylboronic acid,
19) (3aS,4R,6aR)-4-(4-boronobutyl)-1-((1-(cyclohexanecarbonyloxy)ethoxy)carbonyl)octahydropyrrolo[3,4-b]pyrrole-4-carboxylic acid,
20) (3aS,4R,6aR)-4-(4-boronobutyl)-1-((1-(pentanoyloxy)ethoxy)carbonyl)octahydropyrrolo[3,4-b]pyrrole-4-carboxylic acid,
21) (3aS,4R,6aR)-4-(4-boronobutyl)-1-((1-(pivaloyloxy)ethoxy)carbonyl)octahydropyrrolo[2,3-c]pyrrole-4-carboxylic acid,
22) (3aS,4R,6aR)-4-(4-boronobutyl)-1-((1-((S)-2-(dimethylamino)-3-methylbutanoyloxy)ethoxy)carbonyl)octahydropyrrolo[3,4-b]pyrrole-4-carboxylic acid,
23) (3aS,4R,6aR)-4-(4-boronobutyl)-1-((1-(cyclohexanecarbonyloxy)-2-methylpropoxy)carbonyl)octahydropyrrolo[3,4-b]pyrrole-4-carboxylic acid,
24) (3aS,4R,6aR)-4-(4-boronobutyl)-1-((2-methyl-1-(pivaloyloxy)propoxy)carbonyl)octahydropyrrolo[3,4-b]pyrrole-4-carboxylic acid,
25) (3aS,4R,6aR)-4-(4-boronobutyl)-1-(ethoxycarbonyl)octahydropyrrolo[3,4-b]pyrrole-4-carboxylic acid,
26) (3aS,4R,6aR)-4-(4-boronobutyl)-1-(isobutoxycarbonyl)octahydropyrrolo[3,4-b]pyrrole-4-carboxylic acid,
27) (3aS,4R,6aR)-4-(4-boronobutyl)-1-methyloctahydropyrrolo[3,4-b]pyrrole-4-carboxylic acid,
28) (3aS,4R,6aR)-4-(4-boronobutyl)-1-(2-(methylamino)ethyl)octahydropyrrolo[2,3-c]pyrrole-4-carboxylic acid,
29) (3aS,4R,6aR)-4-(4-boronobutyl)-1-(butyryloxymethyl)octahydropyrrolo[3,4-b]pyrrole-4-carboxylic acid,
30) (3aS,4R,6aR)-1-((acetoxymethoxy)carbonyl)-4-(4-boronobutyl)octahydropyrrolo[3,4-b]pyrrole-4-carboxylic acid,
31) (S)-2-((3aS,4R,6aR)-4-(4-boronobutyl)octahydropyrrolo[3,4-b]pyrrole-4-carboxamido)propanoic acid,
32) (S)-2-((3aS,4R,6aR)-4-(4-boronobutyl)octahydropyrrolo[3,4-b]pyrrole-4-carboxamido)-3-methylbutanoic acid,
33) (S)-2-((3aS,4R,6aR)-4-(4-boronobutyl)octahydropyrrolo[2,3-c]pyrrole-4-carboxamido)-4-methylpentanoic

acid,
34) (2S,3R)-2-((3aS,4R,6aR)-4-(4-boronobutyl)octahydropyrrolo[3,4-b]pyrrole-4-carboxamido)-3-methylpentanoic acid,
35) (S)-2-((3aS,4R,6aR)-4-(4-boronobutyl)octahydropyrrolo[3,4-b]pyrrole-4-carboxamido)-3-phenylpropanoic acid,
36) 4-((3aS,4R,6aR)-4-((2-((S)-2-aminopropanoyloxy)ethoxy)carbonyl)octahydropyrrolo[2,3-c]pyrrol-4-yl)butylboronic acid,
37) 4-((3aS,4R,6aR)-4-((2-((S)-2-amino-3-methylbutanoyloxy)ethoxy)carbonyl)octahydropyrrolo[3,4-b]pyrrol-4-yl)butylboronic acid,
38) 4-((3aS,4R,6aR)-4-((2-((2S,3S)-2-amino-3-methylpentanoyloxy)ethoxy)carbonyl)octahydropyrrolo[2,3-c]pyrrol-4-yl)butylboronic acid,
39) 4-((3aS,4R,6aR)-4-((2-((S)-2-amino-4-methylpentanoyloxy)ethoxy)carbonyl)octahydropyrrolo[2,3-c]pyrrol-4-yl)butylboronic acid,
40) 4-((3aS,4R,6aR)-4-((2-((S)-2-amino-3-phenylpropanoyloxy)ethoxy)carbonyl)octahydropyrrolo[2,3-c]pyrrol-4-yl)butylboronic acid,
41) 4-((3aS,4R,6aR)-4-((2-((S)-pyrrolidine-2-carbonyloxy)ethoxy)carbonyl)octahydropyrrolo[3,4-b]pyrrol-4-yl)butylboronic acid,
42) 4-((3aS,4R,6aR)-4-((propionyloxymethoxy)carbonyl)octahydropyrrolo[2,3-c]pyrrol-4-yl)butylboronic acid,
43) 4-((3aS,4R,6aR)-4-((isobutyryloxymethoxy)carbonyl)octahydropyrrolo[3,4-b]pyrrol-4-yl)butylboronic acid,
44) 4-((3aS,4R,6aR)-4-(benzyloxycarbonyl)octahydropyrrolo[3,4-b]pyrrol-4-yl)butylboronic acid,
45) 4-((3aS,4R,6aR)-4-((piperidin-4-ylmethoxy)carbonyl)octahydropyrrolo[3,4-b]pyrrol-4-yl)butylboronic acid,
46) 4-((3aS,4R,6aR)-4-(phenoxycarbonyl)octahydropyrrolo[3,4-b]pyrrol-4-yl)butylboronic acid,
47) 4-((3aS,4R,6aR)-4-((2-methoxyphenoxy)carbonyl)octahydropyrrolo[3,4-b]pyrrol-4-yl)butylboronic acid,
48) (3aS,4R,6aR)-1-((((S)-2-amino-3-phenylpropanoyloxy)methoxy)carbonyl)-4-(4-boronobutyl)octahydropyrrolo[3,4-b]pyrrole-4-carboxylic acid,
49) (3aS,4R,6aR)-1-((((S)-2-aminopropanoyloxy)methoxy)carbonyl)-4-(4-boronobutyl)octahydropyrrolo[3,4-b]pyrrole-4-carboxylic acid,
50) (3aS,4R,6aR)-4-(4-boronobutyl)-1-((((S)-pyrrolidine-2-carbonyloxy)methoxy)carbonyl)octahydropyrrolo[3,4-b]pyrrole-4-carboxylic acid,
51) (3aS,4R,6aR)-1-((((2S,3R)-2-amino-3-hydroxybutanoyloxy)methoxy)carbonyl)-4-(4-boronobutyl)octahydropyrrolo[3,4-b]pyrrole-4-carboxylic acid,
52) (3aS,4R,6aR)-1-((((S)-2-amino-3-methylbutanoyloxy)methoxy)carbonyl)-4-(4-boronobutyl)octahydropyrrolo[3,4-b]pyrrole-4-carboxylic acid,
53) (3aS,4R,6aR)-1-((((S)-2-amino-4-methylpentanoyloxy)methoxy)carbonyl)-4-(4-boronobutyl)octahydropyrrolo[2,3-c]pyrrole-4-carboxylic acid, and
54) (3aS,4R,6aR)-1-((((2S,3S)-2-amino-3-methylpentanoyloxy)methoxy)carbonyl)-4-(4-boronobutyl)octahydropyrrolo[3,4-b]pyrrole-4-carboxylic acid.

**11.** A pharmaceutical composition for the prevention or treatment of cancer or tumors, comprising the compound according to any one of claims 1 to 10, or a pharmaceutically acceptable salt thereof as an active ingredient.

**12.** A compound, which is any one selected from the group consisting of the following:

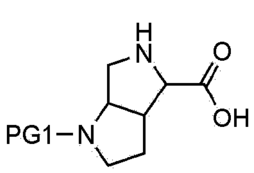
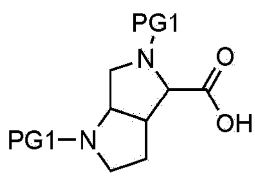
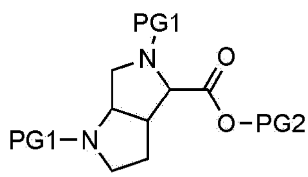

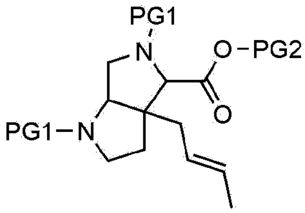
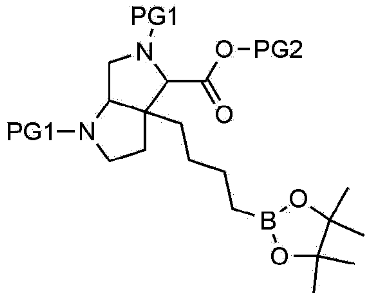

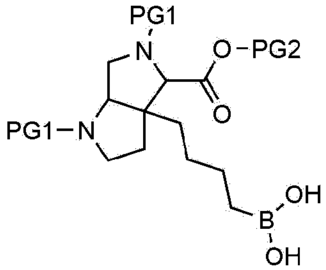
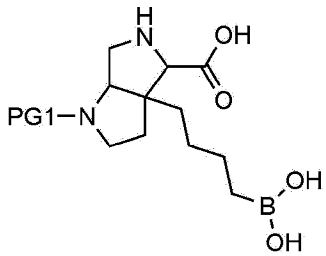

wherein,
PG1 and PG2 are each independently identical or different, and represent a protecting group.

**13.** The compound according to claim 12,
wherein the compound is any one selected from the group consisting of the following:

(3aR,4R,6aR)-1-(tert-butoxycarbonyl)octahydropyrrolo[3,4-b]pyrrole-4-carboxylic acid,
(3aS,4R,6aR)-5-((benzyloxy)carbonyl)-1-(tert-butoxycarbonyl)octahydropyrrolo[3,4-b]pyrrole-4-carboxylic acid,
4,5-dibenzyl 1-(tert-butyl) (3aS,4R,6aR)-hexahydropyrrolo[3,4-b]pyrrole-1,4,5-tricarboxylate,
4,5-dibenzyl 1-(tert-butyl) (3aS,4R,6aR)-4-((E)-but-2-en-1-yl)hexahydropyrrolo[3,4-b]pyrrole-1,4,5-tricarboxylate,
4,5-dibenzyl 1-(tert-butyl)(3aS,4R,6aR)-4-(4-(4,4,5,5-tetramethyl-1,3,2-dioxaborolan-2-yl)butyl)hexahydropyrrolo[3,4-b]pyrrole-1,4,5-tricarboxylate,
(4-((3aS,4R,6aR)-4,5-bis((benzyloxy)carbonyl)-1-(tert-butoxycarbonyl)octahydropyrrolo[3,4-b]pyrrole-4-yl)butyl)boronic acid, and
(3aS,4R,6aR)-5-(benzyloxycarbonyl)-4-(4-boronobutyl)-1-(tert-butoxycarbonyl)octahydropyrrolo[3,4-b]pyrrole-4-carboxylic acid.

## INTERNATIONAL SEARCH REPORT

International application No.
**PCT/KR2023/007804**

**A. CLASSIFICATION OF SUBJECT MATTER**

**C07F 5/02**(2006.01)i; **A61K 31/69**(2006.01)i; **A61P 35/00**(2006.01)i

According to International Patent Classification (IPC) or to both national classification and IPC

**B. FIELDS SEARCHED**

Minimum documentation searched (classification system followed by classification symbols)

C07F 5/02(2006.01); A01N 43/38(2006.01); A61K 31/40(2006.01); A61K 31/407(2006.01); A61K 31/4184(2006.01); A61K 31/69(2006.01); A61P 35/00(2006.01); C07D 401/06(2006.01); C07D 487/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 암(cancer), 아르기나아제(arginase), 옥타하이드로피롤로[3,4-b]피롤(octahydropyrrolo[3,4-b]pyrrole)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2021-141751 A1 (MERCK SHARP & DOHME CORP.) 15 July 2021 (2021-07-15)<br>See claims 1, 20, 21 and 27; examples 1 and 2; and pages 57-60. | 12<br>1-11,13 |
| X | JEANNOTTE, G. et al. Synthesis of fused heteroarylprolines and pyrrolopyrroles. The journal of organic chemistry. 2004, vol. 69, no. 14, pp. 4656-4662.<br>See formula 4. | 12 |
| X | Chemical Abstract Compound. STNext. RN 1888525-39-0 (13 April 2016).<br>See the compounds. | 12 |
| A | WO 2020-131598 A1 (MERCK SHARP & DOHME CORP.) 25 June 2020 (2020-06-25)<br>See entire document. | 1-13 |
| A | WO 2011-068926 A1 (TETRALOGIC PHARMACEUTICALS CORP.) 09 June 2011 (2011-06-09)<br>See entire document. | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 October 2023** | **10 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/007804**

**C. DOCUMENTS CONSIDERED TO BE RELEVANT**

| Category* | Citation of document, with indication, where appropriate, of the relevant passages | Relevant to claim No. |
|---|---|---|
| A | KR 10-2018-0091770 A (DAEWOONG PHARMACEUTICAL CO., LTD.) 16 August 2018 (2018-08-16) See entire document. | 1-13 |

Form PCT/ISA/210 (second sheet) (July 2022)

**INTERNATIONAL SEARCH REPORT**
**Information on patent family members**

International application No.
**PCT/KR2023/007804**

| Patent document cited in search report | Publication date (day/month/year) | Patent family member(s) | Publication date (day/month/year) |
|---|---|---|---|
| WO 2021-141751 A1 | 15 July 2021 | EP 4087583 A1 | 16 November 2022 |
| | | US 2023-0140132 A1 | 04 May 2023 |
| WO 2020-131598 A1 | 25 June 2020 | EP 3897622 A1 | 27 October 2021 |
| | | EP 3897622 A4 | 28 September 2022 |
| | | US 2022-0056051 A1 | 24 February 2022 |
| WO 2011-068926 A1 | 09 June 2011 | None | |
| KR 10-2018-0091770 A | 16 August 2018 | AR 110963 A1 | 22 May 2019 |
| | | AU 2018-218965 A1 | 25 July 2019 |
| | | AU 2018-218965 B2 | 18 June 2020 |
| | | BR 112019016291 A2 | 07 April 2020 |
| | | CA 3049643 A1 | 16 August 2018 |
| | | CA 3049643 C | 06 July 2021 |
| | | CL 2019002019 A1 | 13 December 2019 |
| | | CN 110191882 A | 30 August 2019 |
| | | CN 110191882 B | 13 September 2022 |
| | | CO 2019007834 A2 | 31 July 2019 |
| | | EC SP19051409 A | 31 July 2019 |
| | | EP 3580208 A1 | 18 December 2019 |
| | | EP 3580208 A4 | 26 August 2020 |
| | | EP 3580208 B1 | 01 September 2021 |
| | | ES 2899665 T3 | 14 March 2022 |
| | | JP 2020-506197 A | 27 February 2020 |
| | | JP 6785384 B2 | 18 November 2020 |
| | | KR 10-2084772 B1 | 04 March 2020 |
| | | MA 47469 A | 02 June 2021 |
| | | MA 47469 B1 | 30 November 2021 |
| | | MX 2019009185 A | 26 September 2019 |
| | | MY 196538 A | 19 April 2023 |
| | | NZ 755300 A | 29 October 2021 |
| | | PE 20191477 A1 | 16 October 2019 |
| | | PH 12019501811 A1 | 14 September 2020 |
| | | PL 3580208 T3 | 27 December 2021 |
| | | SG 11201906436 A | 27 August 2019 |
| | | TW 201831463 A | 01 September 2018 |
| | | TW I694070 B | 21 May 2020 |
| | | US 10981917 B2 | 20 April 2021 |
| | | US 2019-0359617 A1 | 28 November 2019 |
| | | WO 2018-147626 A1 | 16 August 2018 |

Form PCT/ISA/210 (patent family annex) (July 2022)